(19) Europäisches Patentamt
European Patent Office
Office européen des brevets

(11) EP 3 686 210 A1

(12) EUROPEAN PATENT APPLICATION

(43) Date of publication:
**29.07.2020 Bulletin 2020/31**

(21) Application number: **19156345.1**

(22) Date of filing: **11.02.2019**

(51) Int Cl.:
***C07H 1/06*** *(2006.01)* ***C07H 1/08*** *(2006.01)*
***C07H 3/06*** *(2006.01)* ***A23L 5/00*** *(2016.01)*
***A23L 29/30*** *(2016.01)* ***B01D 61/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2019 US 201962796272 P**

(71) Applicant: **DuPont Nutrition Biosciences ApS**
**1411 Copenhagen K (DK)**

(72) Inventors:
- **KOIVIKKO, Hannu**
**02460 Kantvik (FI)**
- **JÄRVINEN, Juho**
**02460 Kantvik (FI)**

(74) Representative: **DuPont EMEA**
**Langebrogade 1**
**1411 Copenhagen K (DK)**

# (54) PROCESS FOR PURIFYING A HUMAN MILK OLIGOSACCHARIDE AND RELATED COMPOSITIONS

(57) This specification relates to preparing a purified human milk oligosaccharide (HMO) from an HMO solution by a process comprising nanofiltration, as well as processes for making foods, dietary supplements, medicines and infant formulas comprising a purified HMO. This specification also relates to purified HMOs and foods, dietary supplements, medicines and infant formulas prepared by processes disclosed in this specification.



Printed by Jouve, 75001 PARIS (FR)

## Description

FIELD

This specification relates to preparing a purified human milk oligosaccharide (HMO) from an HMO solution by a process comprising nanofiltration, as well as processes for making foods, dietary supplements, medicines and infant formulas comprising such a purified HMO. This specification also relates to purified HMOs and foods, dietary supplements, medicines and infant formulas prepared by processes disclosed in this specification.

BACKGROUND

Sugars are primary components of human breast milk. Though the disaccharide lactose is predominant amongst these sugars, human milk also contains over 150 sugars having more complex structures known as human milk oligosaccharides (HMOs). While lactose serves as an energy source for infants, HMOs are generally indigestible and provide a variety of other physiological benefits. In particular, many HMOs promote the development of beneficial intestinal microorganisms (the "prebiotic" effect), block adhesion of pathogens to gut epithelial surfaces and modulate the innate immune system. HMOs also are believed to play a role in brain development and neuronal activity. Such benefits make HMOs (such as 2'-fucosyllactose or 3-fucosyllactose) potentially attractive ingredients to be included in food, dietary supplements and medicines, and particularly infant formula.

HMOs include, for example, 2'-fucosyllactose (also referred to as "2'-O-fucosyllactose" or "2'-FL"), 3-fucosyllactose (also referred to as "3-O-fucosyllactose" or "3-FL"), lacto-N-tetraose (also known as "LNT"), 6'-sialyllactose (also referred to as "6'-SL"), 3'-sialyllactose (also referred to as "3'-SL"), lactodifucotetraose, 2',3-difucosyllactose, lacto-N-neotetraose (also referred to as "LNnT"), lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N-triose, lacto-N-neo fucopentaose, lacto-N -neofucopentaose, lacto-N- difucohexaose, 6'-galactosyllactose, 3'-galactosyllactose, lacto-N -hexaose, and lacto-N -neohexaose. Most HMOs in human breast milk are fucosylated, unlike oligosaccharides produced by, for example, dairy animals. The most abundant HMO in human breast milk is 2'-FL.

The development of economically feasible processes for large scale production of HMOs, such as 2'-FL, 3-FL, LNT, 3'-SL and 6'-SL, continues to be a challenge.

Many recent approaches for the synthesis of HMOs involve microbial fermentation processes, which produce HMOs (such as 2'-FL, 3-FL, LNT, 3'-SL and 6'-SL) from lactose. A given HMO is synthesised by cultured microorganisms, such as recombinant *E. coli.* The HMO is then isolated from the broth of biomolecules produced by the culture through a series of purification processes. While there has been success with this approach, the fermentation processes generally produce a complex product mixture which often includes, besides the desired HMOs, other ingredients such as monovalent and divalent salts, lactose, oligosaccharides, monosaccharides, amino acids, polypeptides, proteins, organic acids, nucleic acids, etc. Thus, there continues to be a need for effective, reliable and economically feasible downstream purification processes that provide a useable HMO product.

One of the steps reportedly used in downstream purification processes for HMOs comprises nanofiltration. The membranes used in nanofiltration are often polyamide membranes. The selection of the membrane generally will be dependent on, for example, the molecular weight of the HMO to be separated. 2'-FL and 3-FL, for example, have a molecular weight of about 488 Dalton, while LNT has a molecular weight of about 707 Dalton, and 3'-SL and 6'-SL have a molecular weight of about 633 Dalton. Examples of membranes that have been used for purifying 2'-FL and/or 3-FL include, for example, Suez Desal 5 D-series DK and DL membranes (Suez Water Technologies & Solutions (Trevose, PA)), which have a molecular weight cut-off (also referred to as "MWCO") of from 150 to 300 Dalton to ensure retention of 2'-FL and 3-FL.

Solutions comprising HMOs derived or derivable from the fermentation of microorganisms, such as recombinant *E. coli,* generally contain significant amounts of salts. Furthermore, the concentration of HMOs in these solutions is typically low due to, for example, fermentation needs and conditions used for biomass removal.

Nanofiltration ("NF") has been used to concentrate HMOs (*e.g.,* 2'-FL) and remove some of the salts by retaining 2'-FL in the NF concentrate while allowing the salts to pass through to the permeate. But conventional processes for nanofiltering fermentation products comprising HMOs may produce, for example, concentrates that either have an undesirably high salt concentration or have a low salt concentration but an undesirably low HMO concentration.

The presence of salts in nanofiltration concentrates results in increased costs downstream. For example, additional salt removal steps require additional equipment and typically create of more waste water. Poor salt removal also can lead to formation of unwanted precipitates downstream, which, in turn, can cause fouling in subsequent operational units.

A need continues to exist for more effective, reliable and/or economically feasible processes for producing purified HMOs.

SUMMARY

Briefly, this specification generally provides, in part, a process for preparing a purified HMO from an HMO solution.

In some embodiments, this specification provides, in part, a process for preparing a purified human milk oligosaccharide (HMO). The process comprises:

- feeding an HMO solution into a nanofiltration unit,
- filtrating the HMO solution using the nano filtration unit to produce a concentrate and a permeate, and
- collecting the concentrate.

The HMO solution comprises an aqueous medium comprising an HMO to be purified and a second carbohydrate. And the nanofiltration unit comprises a membrane having:

- an $MgSO_4$ retention of at least about 50% (at a temperature of 25°C, concentration of 2 g/l, pressure of 8 bar and pH of 6-7); and
- an NaCl retention of up to about 60% (at a temperature of 25°C, concentration of 2 g/L, pressure of 8 bar and pH of 6-7).

In some such embodiments, the filtrating is carried out at a temperature of less than about 18°C.

In other such embodiments, the filtrating is carried out at a temperature of less than 10°C.

In other such embodiments, the nanofiltration unit comprises a membrane having an $MgSO_4$ retention of greater than 90% (at a temperature of 25°C, concentration of 2 g/L, pressure of 8 bar and pH of 6-7).

In other such embodiments, the nanofiltration unit comprises a membrane having a molecular weight cut off of less than 600 Dalton.

In other such embodiments, the membrane has a lactose retention of at least about 80% (at a temperature of 25°C, concentration of 20g/L, flux of 5-20 $kg/m^2/h$, and pH of 8).

In other such embodiments, the process further comprises diafiltration, and the process comprises feeding into the nanofiltration unit water for diafiltration in addition to the HMO solution. In some such embodiments, the weight ratio of the water for diafiltration to the HMO solution is less than 1.6 when normalized to an HMO solution having a dry substance composition of 4% (w/w).

In some embodiments comprising diafiltration, the weight ratio of the water for diafiltration to the HMO solution is from about 0.09 to 0.3 when normalized to an HMO solution having a dry substance composition of 4% (w/w). In some such embodiments, the nanofiltration provides a salt removal of at least about 40%. In other such embodiments, the nanofiltration, the nanofiltration additionally or alternatively provides a yield of the purified HMO of at least about 85%.

In other embodiments comprising diafiltration, the weight ratio of the water for diafiltration to the HMO solution is from 0.3 to 0.6 when normalized to an HMO solution having a dry substance composition of 4% (w/w). In some such embodiments, the nanofiltration provides a salt removal of at least about 90%. In other such embodiments, the nanofiltration, the nanofiltration additionally or alternatively provides a yield of the purified HMO of at least about 90%.

In other embodiments comprising diafiltration, the weight ratio of the water for diafiltration to the HMO solution is from 0.6 to 0.9 when normalized to an HMO solution having a dry substance composition of 4% (w/w). In some such embodiments, the nanofiltration provides a salt removal of at least 68%. In other such embodiments, the nanofiltration, the nanofiltration additionally or alternatively provides a yield of the purified HMO of at least about 85%.

In some embodiments, this specification provides, in part, a purified HMO (or purified mixture of HMOs) prepared by a process of this specification.

In some embodiments, this specification provides, in part, a process for making a food, dietary supplement or medicine. The process comprises preparing a purified HMO (or purified mixture of HMOs) according to the above process, and mixing the purified HMO (or mixture) with an ingredient suitable for the food, dietary supplement or medicine.

In some embodiments, this specification provides, in part, a food, dietary supplement or medicine prepared by a process of this specification.

In some embodiments, this specification provides, in part, a process for making an infant formula. The process comprises preparing a purified HMO (or purified mixture of HMOs) according to the above process, and mixing the purified HMO (or mixture) with an ingredient suitable for an infant formula.

In some embodiments, this specification provides, in part, an infant formula prepared by a process of this specification.

Further benefits of the teachings of this specification will be apparent to one skilled in the art from reading this specification.

BRIEF DESCRIPTION OF THE FIGURES

**FIG. 1** shows $MgSO_4$ retentions for DK, NFX, DL, NFW, NF245, XN45, XN45 (old), NDX, UA60 and NFG membranes at different temperatures.
**FIG. 2** shows NaCl retentions for DK, NFX, DL, NFW, NF245, XN45, XN45 (old), NDX, UA60, and NFG membranes at different temperatures.

DETAILED DESCRIPTION

This detailed description is intended to acquaint others skilled in the art with Applicant's invention, its principles, and its practical application so that others skilled in the art may adapt and apply Applicant's invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.
This specification provides a process for preparing a purified human milk oligosaccharide (HMO). The process comprises:

- feeding an HMO solution into a nanofiltration unit,
- filtrating the HMO solution using the nanofiltration unit to produce a concentrate and a permeate, and
- collecting the concentrate.

The HMO solution comprises an aqueous medium, which comprises both an HMO to be purified and another carbohydrate. Typically, the aqueous medium further comprises other ingredients, such as, for example, one or more additional carbohydrates, which may include one or more other HMOs and/or one or more various other carbohydrates.

**Human Milk Oligosaccharides**

There are over 150 known human milk oligosaccharides generally present in human breast milk. A process of this invention may be used to prepare a single purified HMO or a purified mixture of two or more HMOs.
In some embodiments, a process of this specification comprises preparing a purified HMO selected from fucosyllactoses (such as 2'-fucosyllactose (also referred to as "2'-FL") or 3-fucosylslactose (also referred to as "3-FL")), sialyllactoses (such as 3'-silayllactose (also referred to as "N-acetylneuraminyl-2-3-galactopyranosyl-1-4-glucopyranose" or "3'-SL") or 6'-sialyllactose (also referred to as "6'-SL")), lacto-N-tetraose (also referred to as "LNT"), lactodifucotetraose, difucosyllactose (also referred to as "DiFL"), lacto-N-neotetraose (also referred to as "LNnT"), lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N-triose, lacto-N-neo fucopentaose, lacto-N -neofucopentaose, lacto-N- difucohexaose, 6'-galactosyllactose, 3'-galactosyllactose, lacto-N -hexaose, or lacto-N -neohexaose. In some embodiments, a process of this specification comprises preparing a purified HMO mixture comprising one or more of the above-listed HMOs. In some embodiments, a process of this specification comprises preparing a purified HMO mixture comprising at least two of the above-listed HMOs.
In some embodiments, a process of this specification is used to prepare a purified HMO selected from a fucosyllactose (*e.g.,* 2'-FL or 3-FL), a sialyllactose (*e.g.,* 3'-SL or 6'-SL), lacto-N-tetraose.
In some embodiments, a process of this specification is used to prepare a purified fucosyllactose (also referred to as "FL"). At room temperature and pressure, a fucosyllactose is typically a white to ivory colored solid and soluble in water. In some embodiments, the purified fucosyllactose is 2'-FL. In some embodiments, the purified fucosyllactose is 3-FL. In some embodiments, a process of this specification is used to prepare a purified HMO mixture comprising a fucosyllactose. In some embodiments, a process of this specification is used to prepare a purified HMO mixture comprising 2'-FL or 3-FL. In some embodiments, a process of this specification is used to prepare a purified HMO mixture comprising at least two fucosyllactoses. In some embodiments, a process of this specification is used to prepare a purified HMO mixture comprising 2'-FL and 3-FL.
In some embodiments, a process of this specification comprises preparing a purified sialyllactose (also referred to as "SL"). In some embodiments, the purified sialyllactose is 3'-SL. In some embodiments, the purified sialyllactose is 6'-SL". In some embodiments, a process of this specification is used to make a purified HMO mixture comprising a sialyllactose. In some embodiments, a process of this specification is used to prepare a purified HMO mixture comprising 3'-SL or 6'-SL. In some embodiments, a process of this specification is used to prepare a purified HMO mixture comprising at least two sialyllactoses. In some embodiments, a process of this specification is used to prepare a purified HMO mixture comprising 3'-SL and 6'-SL.

In some embodiments, a process of this specification comprises preparing purified lacto-N-tetraose ("LNT"). In some embodiments, the process of this specification is used to make a purified HMO mixture comprising LNT.

**HMO Solution**

An "HMO solution" from which an HMO is purified in accordance with this specification comprises an aqueous medium. The aqueous medium comprises both the HMO and a second carbohydrate.

In some embodiments, the aqueous medium is water.

In some embodiments, the HMO is selected from 2'-FL, 3-FL, LNT, 6'-SL, 3'-SL, lactodifucotetraose, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N-triose, lacto-N-neo fucopentaose, lacto-N -neofucopentaose, lacto-N- difucohexaose, 6'-galactosyllactose, 3'-galactosyllactose, lacto-N -hexaose, and lacto-N -neohexaose.

In some embodiments, the HMO is a fucosyllactose.

In some embodiments, the HMO is 2'-FL.

In some embodiments, the HMO is 3-FL.

In some embodiments, the HMO is a sialyllactose.

In some embodiments, the HMO is 3'-SL.

In some embodiments, the HMO is 6'-SL.

In some embodiments, the HMO is LNT.

In some embodiments, the second carbohydrate is an HMO.

In some embodiments, the second carbohydrate is selected from 2'-FL, 3-FL, LNT, 6'-SL, 3'-SL, lactodifucotetraose, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N -triose, lacto-N-neo fucopentaose, lacto-N -neofucopentaose, lacto-N-difucohexaose, 6'-galactosyllactose, 3'-galactosyllactose, lacto-N -hexaose, and lacto-N-neohexaose.

In some embodiments, the second carbohydrate is fucosyllactose.

In some embodiments, the second carbohydrate is 2'-FL.

In some embodiments, the second carbohydrate is 3-FL.

In some embodiments, the second carbohydrate is a sialyllactose.

In some embodiments, the second carbohydrate is 3'-SL.

In some embodiments, the second carbohydrate is 6'-SL.

In some embodiments, the second carbohydrate is LNT.

In some embodiments, the second carbohydrate is lactose, fucose, 2',3-di-O-fucosyllactose, 2'-O-fucosyl lactulose, lactulose, glucose or galactose.

In some embodiments, the second carbohydrate is lactose.

In some embodiments, the second carbohydrate is fucose.

In some embodiments, the second carbohydrate is 2',3-di-O-fucosyllactose.

In some embodiments, the second carbohydrate is 2'-O-fucosyl lactulose.

In some embodiments, the second carbohydrate is lactulose.

In some embodiments, the second carbohydrate is glucose.

In some embodiments, the second carbohydrate is galactose.

In some embodiments, the HMO solution comprises at least two HMOs. In some embodiments, the HMO solution comprises at least three HMOs. In some embodiments, the HMO solution comprises at least four HMOs. In some embodiments, the HMO solution comprises at least five HMOs.

In some embodiments, the HMO solution comprises two or more HMOs selected from fucosyllactoses, sialyllactoses and LNT. In some such embodiments, the fucosyllactoses are selected from 2'-FL and 3-FL and the sialyllactoses are selected from 3'-SL and 6'-SL.

In some embodiments, the HMO solution comprises 2'-FL and 3-FL.

In some embodiments, the HMO solution comprises 3'-SL and 6'-SL.

Typically, the HMO solution further comprises one or more ingredients in addition to the HMO to be purified and the second carbohydrate. Such other ingredients may include, for example, monovalent and divalent salts, lactose, oligosaccharides, monosaccharides, amino acids, polypeptides, proteins, organic acids, nucleic acids, etc.

In some embodiments, the HMO solution comprises (in addition to the HMO to be purified and the second carbohydrate) one or more additional HMOs and/or one or more other types of carbohydrates.

In some embodiments, the HMO solution comprises (in addition to the HMO to be purified and the second carbohydrate) one or more oligosaccharides.

In some embodiments, the HMO solution comprises (in addition to the HMO to be purified and the second carbohydrate) one or more additional HMOs.

In some embodiments, the HMO solution comprises (in addition to the HMO to be purified and the second carbohydrate) one or more additional HMOs selected from 2'-FL, 3-FL, LNT, 6'-SL, 3'-SL, lactodifucotetraose, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N -triose, lacto-N-neo fucopentaose, lacto-N -neofucopentaose, lacto-N-difucohexaose, 6'-galactosyllactose, 3'-galactosyllactose, lacto-N -hexaose, and lacto-N-neohexaose.

In some embodiments, the HMO solution comprises (in addition to the HMO to be purified and the second carbohydrate) 2'-O-fucosyl lactulose.

In some embodiments, the HMO solution comprises (in addition to the HMO to be purified and the second carbohydrate) DiFL.

In some embodiments, the HMO solution comprises (in addition to the HMO to be purified and the second carbohydrate) lactose.

In some embodiments, the HMO solution comprises (in addition to the HMO to be purified and the second carbohydrate) lactulose.

In some embodiments, the HMO solution comprises (in addition to the HMO to be purified and the second carbohydrate) one or more monosaccharides.

In some embodiments, the HMO solution comprises (in addition to the HMO to be purified and the second carbohydrate) fucose.

In some embodiments, the HMO solution comprises (in addition to the HMO to be purified and the second carbohydrate) glucose.

In some embodiments, the HMO solution comprises (in addition to the HMO to be purified and the second carbohydrate) galactose.

In some embodiments, the HMO solution comprises (in addition to the HMO to be purified and the second carbohydrate) one or more monovalent salts.

In some embodiments, the HMO solution comprises (in addition to the HMO to be purified and the second carbohydrate) one or more divalent salts.

In some embodiments, the HMO solution comprises (in addition to the HMO to be purified and the second carbohydrate) one or more amino acids.

In some embodiments, the HMO solution comprises (in addition to the HMO to be purified and the second carbohydrate) one or more proteins.

In some embodiments, the HMO solution comprises (in addition to the HMO to be purified and the second carbohydrate) one or more organic acids.

In some embodiments, the HMO solution comprises (in addition to the HMO to be purified and the second carbohydrate) one or more nucleic acids.

In some embodiments, the HMO solution comprises (or is derived in whole or in part from) a natural source of the HMO to be purified, such as, for example, an animal milk (e.g., human milk).

In some embodiments, the HMO solution comprises (or is derived in whole or in part from) the product of a chemical synthesis. In some embodiments, the HMO solution comprises (or is derived in whole or in part from) the product of a chemical synthesis of the HMO to be purified. In some embodiments, the other carbohydrate(s) in the solution comprise carbohydrate reagents used in the synthesis, carbohydrate components of the medium used in the synthesis and/or carbohydrates formed during and/or after the synthesis.

In some embodiments, the HMO solution comprises (or is derived in whole or in part from) a product of a fermentation. In some such embodiments, the HMO solution is (or derived in whole or in part from) the product of a fermentation used to make the HMO to be purified. In some such embodiments, the other carbohydrate(s) in the solution is/are from the culture medium used in the fermentation and/or formed during and/or after the fermentation. In some embodiments, the fermentation comprises culturing, in an aqueous culture medium comprising a carbohydrate (such as lactose and/or fucose), a recombinant microorganism comprising at least one recombinant polynucleotide sequence encoding an enzyme capable of producing an HMO. The product of the fermentation process may be referred to as a fermentation "product" or "broth." The product typically comprises many ingredients in addition to the HMO to be purified, including, for example, monovalent and divalent salts, lactose, oligosaccharides, monosaccharides, amino acids, polypeptides, proteins, organic acids, nucleic acids, etc.

Examples of enzymes often useful for producing an HMO include fucosyltransferases (such as $\alpha$-1,2-fucosyl transferases (EC 2.4.1.69), $\alpha$-1,3-fucosyl transferases, 3-galactosyl-N-acetylglucosaminide 4-alpha-L-fucosyltransferase (EC 2.4.1.65), 4-galactosyl-N-acetylglucosaminide 3-alpha-L-fucosyltransferase (EC 2.4.1.152), peptide-O-fucosyltransferase (EC 2.4.1.221)), glycosyltransferases (such as GT1, GT2, GT10 GT11, GT23, GT37, GT65, GT68, and/or GT74) and sialyltransferases (EC 2.4.99.-). The enzymes capable of producing an HMO may originate from, but are not limited to, *Helicobacter pylori, Campylobacter jejuni, Dictyostellium discoideum, Mus musculus,* and *Homo sapiens.*

In some embodiments, the HMO to be purified is a fucosyllactose, and the HMO solution comprises (or is derived in whole or in part from) a product of a fermentation process wherein the fermentation process comprises culturing, in an aqueous culture medium comprising a carbohydrate (such as lactose and/or fucose), a recombinant microorganism comprising a recombinant polynucleotide sequence encoding an $\alpha$-1,2-fucosyl transferase (EC 2.4.1.69) or $\alpha$-1,3-fucosyl transferase (EC 2.4.1.214).

In general, when the HMO solution comprises (or is derived in whole or in part from) a product of a fermentation process, the process of this specification comprises one or more process steps wherein the cell biomass of the microorganisms used in the fermentation are separated from the fermentation product. Typically, this occurs before nanofiltration.

Cell biomass may be separated from a fermentation product using, for example, filtration, centrifugation, sedimentation and/or other process suitable for removing cell biomass.

In some embodiments, separation of microorganisms from a fermentation product comprises ultrafiltration (also referred to as "UF"). Ultrafiltration can also be particularly beneficial to, for example, remove large biomolecules, such as endotoxins, proteins, nucleic acids and lipopolysaccharides.

In some embodiments, the ultrafiltration is carried out using a cross-flow filter. Typically, the ultrafiltration membrane pore size is from about 0.1 to 0.001 micron.

In some embodiments, the ultrafiltration is carried out for at least about 1 day. In some embodiments, the ultrafiltration is carried out for no greater than about 5 days. In some embodiments, the ultrafiltration is carried out for from about 1 day to about 5 days. In some embodiments, the ultrafiltration is carried out for from 1 day to 4 days. In some embodiments, the ultrafiltration is carried out for from 1 day to 3 days. In some embodiments, the ultrafiltration is carried out for from 1 to 48 hr. In some embodiments, the ultrafiltration is carried out for from 3 to 48 hr. In some embodiments, the ultrafiltration is carried out for from 6 to 48 hr. In some embodiments, the ultrafiltration is carried out for from 9 to 48 hr. In some embodiments, the ultrafiltration is carried out for from 1 to 24 hr. In some embodiments, the ultrafiltration is carried out for 3 to 24 hr. In some embodiments, the ultrafiltration is carried out for 6 to 24 hr. In some embodiments, the ultrafiltration is carried out for 9 to 24 hr. In some embodiments, the ultrafiltration is carried out for 1 to 18 hr. In some embodiments, the ultrafiltration is carried out for 3 to 18 hr. In some embodiments, the ultrafiltration is carried out for from 6 to 18 hr. In some embodiments, the ultrafiltration is carried out for from 9 to 18 hr. In some embodiments, the ultrafiltration is carried out for from 1 to 15 hr. In some embodiments, the ultrafiltration is carried out for from 3 to 15 hr. In some embodiments, the ultrafiltration is carried out for from 6 to 15 hr. In some embodiments, the ultrafiltration is carried out for from 9 to 15 hr. In some embodiments, the ultrafiltration is carried out for from 1 to 12 hr. In some embodiments, the ultrafiltration is carried out for from 3 to 12 hr. In some embodiments, the ultrafiltration is carried out for from 6 to 12 hr. In some embodiments, the ultrafiltration is carried out for from 9 to 12 hr. In some embodiments, the ultrafiltration is carried out for from 1 to 9 hr. In some embodiments, the ultrafiltration is carried out for from 3 to 9 hr. In some embodiments, the ultrafiltration is carried out for from 6 to 9 hr. In some embodiments, the ultrafiltration is carried out for about 9 hr. In some embodiments, the ultrafiltration is carried out for about 12 hr.

In some embodiments, the ultrafiltration is carried out at a temperature of no greater than about 18°C or less. In some embodiments, the ultrafiltration is carried out at a temperature of no greater than 16°C. In some embodiments, the ultrafiltration is carried out at a temperature of less than 16°C. In some embodiments, the ultrafiltration is carried out at a temperature of no greater than 15°C. In some embodiments, the ultrafiltration is carried out at a temperature of less than 15°C. In some embodiments, the ultrafiltration is carried out at a temperature of no greater than 10°C. In some embodiments, the ultrafiltration is carried out at a temperature of less than 10°C.

In some embodiments, the ultrafiltration is carried out at a temperature from 2 to 18°C. In some embodiments, the ultrafiltration is carried out at a temperature of from 4 to 16°C. In some embodiments, the ultrafiltration is carried out at a temperature of from 2 to 16°C. In some embodiments, the ultrafiltration is carried out at a temperature of from 5 to 15°C. In some embodiments, the ultrafiltration is carried out at a temperature of from 2 to 10°C.

In some embodiments, the ultrafiltration is carried out at a temperature of from 2 to 18°C for from about 3 to about 12 hr. In some embodiments, the ultrafiltration is carried out at a temperature of from 2 to 10°C for from about 1 to about 48 hr.

In some embodiments, the ultrafiltration is carried out at a temperature of less than 18°C for from about 3 to about 12 hr. In some embodiments, the ultrafiltration is carried out at a temperature of less than 10°C for from about 1 to about 48 hr.

Carrying out ultrafiltration at a temperature of no greater than 18°C can be helpful to, for example, improve microbiological stability. The term "improve microbiological stability," as used here, refers to less or no growth of microorganisms in the solution when compared to microorganism growth that would occur if the ultrafiltration is carried out at a greater temperature (e.g., >20°C), when all other factors are the same.

**Nanofiltration**

Nanofiltration is generally a pressure-driven membrane filtration-based process. Nanofiltration membranes have pore sizes which are generally smaller than those used in microfiltration and ultrafiltration. The membrane can be, for example, tubular, spiral or flat in shape.
Nano filtration can concentrate an HMO in an HMO solution by, for example, reducing the volume (*e.g.*,, by removing water) while also removing salts and various small biomolecules and other impurities.
This specification provides, in part, a process for purifying a human milk oligosaccharide from an HMO solution. The process comprises:

- feeding the HMO solution into a nanofiltration unit,
- filtrating the HMO solution using the nanofiltration unit to produce a concentrate and a permeate, and
- collecting the concentrate.

In this process, the HMO solution comprises an HMO and another carbohydrate that are both in an aqueous medium.
The nanofiltration produces a concentrate that is enriched in an HMO as compared to the HMO solution feed. In some such embodiments, the concentrate is further enriched in at least one additional HMO as well.
In some embodiments, the nanofiltration concentrate is enriched in an HMO selected from fucosyllactoses (such as 2'-FL or 3-FL), sialyllactoses (such as 3'-SL or 6'-SL), LNT, lactodifucotetraose, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N-triose, lacto-N-neo fucopentaose, lacto-N -neofucopentaose, lacto-N- difucohexaose, 6'-galactosyllactose, 3'-galactosyllactose, lacto-N -hexaose, and lacto-N -neohexaose.
In some embodiments, the nanofiltration concentrate is enriched in a fucosyllactose.
In some embodiments, the nanofiltration concentrate is enriched in 2'-FL.
In some embodiments, the nanofiltration concentrate is enriched in 3-FL.
In some embodiments, the nanofiltration concentrate is enriched in a sialyllactose.
In some embodiments, the nanofiltration concentrate is enriched in 3'-SL.
In some embodiments, the nanofiltration concentrate is enriched in 6'-SL.
In some embodiments, the nanofiltration concentrate is enriched in LNT.
In some embodiments, the nanofiltration concentrate of an embodiment discussed above is further enriched in at least one additional HMO during the nanofiltration. In some such embodiments, the additional HMO(s) is/are selected from fucosyllactoses (such as 2'-FL or 3-FL), sialyllactoses (such as 3'-SL or 6'SL), LNT, lactodifucotetraose, DiFL, LNnT, lacto-N-fucopentaose, lacto-N-difucohexaose, lacto-N-neodifucohexaose, lacto-N-neooctaose, lacto-N-fucopentaose, lacto-N-neofucopentaose, 3'sialyl-3-fucosyllactose, sialyl-lacto-N-tetraose, LS-tetrasaccharide, 3'-sialyllactose, lacto-N -triose, lacto-N-neo fucopentaose, lacto-N - neofucopentaose, lacto-N- difucohexaose, 6'-galactosyllactose, 3'-galactosyllactose, lacto-N - hexaose, and lacto-N -neohexaose.
The "HMO retention" during nano filtration is the percentage of an HMO fed into the nanofiltration unit that is retained in the nanofiltration concentrate.
In some embodiments, the HMO retention is increased by using a more diluted HMO solution and/or increasing the flux. Alternatively, in some embodiments, the HMO retention is decreased by using a more concentrated HMO solution and/or decreasing the flux.
In some embodiments, the nanofiltration unit is characterized as having an HMO retention of the desired HMO of at least about 96.0% when operating at an average flux of from 5 to 20 kg/$m^2$/hr and a concentration of the HMO of from 50 to 300 g/L. In some such embodiments, the retention is from about 96.0% to about 99.9%. In other such embodiments, the retention is from 96.0 to 99.8%. In other such embodiments, the retention is from 96.0 to 99.7%. In other such embodiments, the retention is at least 96.5%. In other such embodiments, the retention is from 96.5 to 99.8%. In other such embodiments, the retention is from 96.5 to 99.7%. In other such embodiments, the retention is at least 97.0%. In other such embodiments, the retention is from 97.0 to 99.8%. In other such embodiments, the retention is from 97.0 to 99.7%. In other such embodiments, the retention is from 97.0 to 99.5%. In other such embodiments, the retention is from 97.3% to 99.8%. In other such embodiments, the retention is from 97.5 to 99.8%. In other such embodiments, the retention is at least 98.0%. In other such embodiments, the retention is at least 99.0%. In other such embodiments, the retention is from 99.0 to 99.8%. In other such embodiments, the retention is from 99.0% to 99.7%. In other such embodiments, the retention is from 99.0 to 99.5%. In other such embodiments, the retention is at least 99.3%. In other such embodiments, the retention is from 99.3 to 99.8%. In other such embodiments, the retention is at least 99.5%. In other such embodiments, the retention is from 99.5 to 99.8%. In other such embodiments, the retention is at least 99.6%. In other such embodiments, the retention is at least 99.7%. In other such embodiments, the retention is at least 99.8%.

In some embodiments, the nanofiltration unit is characterized as providing a cumulative yield (including from any diafiltration) of the desired HMO of at least about 85.0%. In some such embodiments, the yield is from 85% to 99.9%. In other such embodiments, the yield is at least 87%. In other such embodiments, the yield is from 87% to 99.9%. In other such embodiments, the yield is at least 90%. In other such embodiments, the yield is from 90% to 99.9%. In other such embodiments, the yield is from 90% to 98.0%. In other such embodiments, the yield is greater than 90%. In other such embodiments, the yield is greater than 90% and no greater than 98.0%. In other such embodiments, the yield is at least 92%. In other such embodiments, the yield is from 92% to 99.9%. In other such embodiments, the yield is at least 95%. In other such embodiments, the yield is from 95% to 99.9%. In other such embodiments, the yield is at least about 96.0%. In some such embodiments, the yield is from about 96.0% to about 99.8%. In other such embodiments, the yield is from 96.0 to 99.8%. In other such embodiments, the yield is from 96.0 to 99.7%. In other such embodiments, the yield is at least 96.5%. In other such embodiments, the yield is from 96.5 to 99.8%. In other such embodiments, the yield is from 96.5 to 99.7%. In other such embodiments, the yield is at least 97.0%. In other such embodiments, the yield is from 97.0 to 99.8%. In other such embodiments, the yield is from 97.0 to 99.7%. In other such embodiments, the yield is from 97.0 to 99.5%. In other such embodiments, the yield is from 97.3% to 99.8%. In other such embodiments, the yield is from 97.5 to 99.8%. In other such embodiments, the yield is at least 98.0%. In other such embodiments, the yield is at least 99.0%. In other such embodiments, the yield is from 99.0 to 99.8%. In other such embodiments, the yield is from 99.0% to 99.7%. In other such embodiments, the yield is from 99.0 to 99.5%.

The conductivity of a solution, such as the HMO solution, the nanofiltration permeate and the nanofiltration concentrate, can be determined by measuring the ability of the solution to conduct electricity. A variety of probes useful for measuring conductivity are commercially available.

The "conductivity retention" during nano filtration is calculated as the percentage of the conductivity of the solution fed into the nanofiltration unit that is retained by the nanofiltration concentrate.

In some embodiments, the nanofiltration unit is characterized as having a conductivity retention of no greater than about 90% when operating at an average flux of from 5 to 20 kg/$m^2$/hr and a concentration of the desired HMO of from 50 to 300 g/L. In some such embodiments, the conductivity retention is no greater than 85%. In other such embodiments, the conductivity retention is no greater than 83%. In other such embodiments, the conductivity retention is no greater than 80%. In other such embodiments, the conductivity retention is no greater than 79%. In other such embodiments, the conductivity retention is no greater than 75%. In other such embodiments, the conductivity retention is no greater than 70%. In other such embodiments, the conductivity retention is no greater than 67%. In other such embodiments, the conductivity retention is no greater than 65%. In other such embodiments, the conductivity retention is no greater than 60%. In other such embodiments, the conductivity retention is no greater than 55%. In other such embodiments, the conductivity retention is no greater than 50%.

The presence of multivalent salts (e.g., phosphates and sulfates) tends to lead to greater conductivity retentions than monovalent salts (e.g., chlorides and acetates). Conductivity retentions also tend to be greater at higher pH's, particularly when multivalent salts are present. Without being bound by any particular theory, it is believed this is due, at least in part, to multivalent salts converting to monovalent salts at lower pH's.

In some embodiments, the nanofiltration unit is characterized as having an HMO retention of the desired HMO of at least about 96.0% and a conductivity retention of no greater than about 90% when operating at an average flux of from 5 to 20 kg/$m^2$/hr and a concentration of the HMO of from 50 to 300 g/L. In some such embodiments, the HMO retention of the desired HMO is at least 99.0% and the conductivity retention of no greater than 70%. In other such embodiments, the HMO retention of the desired HMO is from 99.0% to 99.7% and the conductivity retention of no greater than 70%. In other such embodiments, the HMO retention of the desired HMO is greater than 99.0% and the conductivity retention of no greater than 70%. In other such embodiments, the HMO retention of the desired HMO is greater than 99.0% and the conductivity retention of no greater than 85%. In other such embodiments, the HMO retention is greater than 99.3% and the conductivity retention of no greater than 85%.In other such embodiments, the HMO retention is greater than 99.7% and the conductivity retention of no greater than 83%.

The term "salt removal" as used in the description refers to the amount of salt removed from an HMO solution during nanofiltration. The salt removal can be calculated using either of the following equations:

$$\text{Salt Removal} = \frac{m_{permeate} \text{ x } conductivity_{permeate}}{m_{feed} \text{ x } conductivity_{feed}} \text{ x } 100\%$$

or:

$$\text{Salt Removal} = \left( 1 - \frac{m_{concentrate} \times conductivity_{concentrate}}{m_{feed} \times conductivity_{feed}} \right) \times 100\%$$

The term "m" refers to mass, and the term "feed" refers to the HMO solution which is fed into the nanofiltration unit.

In some embodiments, the nanofiltration unit is characterized as having a salt removal of at least about 40%. In some embodiments, the salt removal is from about 40% to about 96% when operating at an average flux of from 5 to 20 kg/m$^2$/hr and a concentration of the HMO of from 50 to 300 g/L. In some such embodiments, the salt removal is from 40% to 96%. In other such embodiments, the salt removal is at least 50%. In other such embodiments, the salt removal is from 50% to 96%. In other such embodiments, the salt removal is at least about 60%. In other such embodiments, the salt removal is from 60% to 96%. In other such embodiments, the salt removal is least 68%. In other such embodiments, the salt removal is from 68% to 96%. In other such embodiments, the salt removal is at least 69%. In other such embodiments, the salt removal is from 69% to 96%. In other such embodiments, the salt removal is least 70%. In other such embodiments, the salt removal is from 70% to 96%. In other such embodiments, the salt removal is least 75%. In other such embodiments, the salt removal is from 75% to 96%. In other such embodiments, the salt removal is least 80%. In other such embodiments, the salt removal is from 80% to 96%. In other such embodiments, the salt removal is least 85%. In other such embodiments, the salt removal is from 85% to 96%. In other such embodiments, the salt removal is at least 90%. In other such embodiments, the salt removal is from 90% to 96%. In other such embodiments, the salt removal is at least 92%. In other such embodiments, the salt removal is from 92% to 96%. In other such embodiments, the salt removal is at least 95%. In other such embodiments, the salt removal is from 95% to 96%.

The presence of multivalent salts (e.g., phosphates and sulfates) tends to lead to greater salt removal than monovalent salts (e.g., chlorides and acetates). Salt removal also tends to be greater at higher pH's, particularly when multivalent salts are present. Without being bound by any particular theory, it is believed this is due, at least in part, to multivalent salts converting to monovalent salts at lower pH's.

In some embodiments, the nanofiltration unit is characterized as having an HMO retention of the desired HMO of at least about 85.0% and a salt removal of at least about 40% when operating at an average flux of from 5 to 20 kg/m$^2$/hr and a concentration of the HMO of from 50 to 300 g/L. In some such embodiments, the HMO retention of the desired HMO is from about 85% to 99.9% and the salt removal is from 40% to 96%. In other such embodiments, the HMO retention of the desired HMO is from about 85% to 99.9% and the salt removal is from 68% to 96%. In other such embodiments, the HMO retention of the desired HMO is from about 85% to 99.9% and the salt removal is from 69% to 96%. In other such embodiments, the HMO retention of the desired HMO is from about 85% to 99.9% and the salt removal is from 70% to 96%. In other such embodiments, the HMO retention of the desired HMO is from about 85% to 99.9% and the salt removal is from 75% to 96%. In other such embodiments, the HMO retention of the desired HMO is from about 90% to 99.9% and the salt removal is from 90% to 96%.

In some embodiments, the nanofiltration comprises diafiltration. In some such embodiments, the diafiltration comprises adding a solvent to a feed stream of the nanofiltration unit. In some embodiments, the solvent is water.

In some embodiments wherein the nanofiltration comprises diafiltration, wherein solvent (e.g., water) is fed into the nanofiltration unit with the HMO solution, and the weight ratio of the solvent to the HMO solution fed into the nanofiltration unit is less than 1.6 (normalized to an HMO solution having a dry substance composition of 4% (w/w)). In some such embodiments, the weight ratio of the solvent to the HMO solution fed into the nanofiltration unit is from about 0.09 to about 1.5. In some such embodiments, the weight ratio of the solvent to the HMO solution fed into the nanofiltration unit is from 0.09 to 1.5. In some such embodiments, the weight ratio of the solvent to the HMO solution fed into the nanofiltration unit is from 0.3 to 1.5. In some such embodiments, the weight ratio of the solvent to the HMO solution fed into the nanofiltration unit is from 0.6 to 1.5. In some such embodiments, the weight ratio of the solvent to the HMO solution fed into the nanofiltration unit is from 0.9 to 1.5. In some such embodiments, the weight ratio of the solvent to the HMO solution fed into the nanofiltration unit is from 1.0 to 1.5. In other such embodiments, the weight ratio of the solvent to the HMO solution fed into the nanofiltration unit is less than 1.5. In other such embodiments, the weight ratio of the solvent to the HMO solution fed into the nanofiltration unit is no greater than 1.0. In other such embodiments, the weight ratio of the solvent to the HMO solution fed into the nanofiltration unit is from 0.09 to 1.0. In other such embodiments, the weight ratio of the solvent to the HMO solution fed into the nanofiltration unit is from 0.3 to 1.0. In other such embodiments, the weight ratio of the solvent to the HMO solution fed into the nanofiltration unit is from 0.6 to 1.0. In other such embodiments, the weight ratio of the solvent to the HMO solution fed into the nanofiltration unit is from 0.75 to 1.0. In other such embodiments, the weight ratio of the solvent to the HMO solution fed into the nanofiltration unit is no greater than 0.9. In other such embodiments, the weight ratio of the solvent to the HMO solution fed into the nanofiltration unit is from 0.09 to 0.9. In other such embodiments, the weight ratio of the solvent to the HMO solution fed into the nanofiltration unit is from 0.3 to 0.9. In other such embodiments, the weight ratio of the solvent to the HMO solution fed into the nanofiltration unit is from 0.6 to 0.9. In other such embodiments, the weight ratio of the solvent to the HMO solution fed into the nanofiltration unit is no greater than 0.75. In other such embodiments, the weight ratio of the solvent to the HMO solution fed into the nanofiltration unit is from 0.09 to 0.75. In other such embodiments, the weight ratio of the solvent to the HMO solution fed into the nanofiltration unit is from 0.3 to 0.75. In other such embodiments, the weight ratio of the solvent to the HMO solution fed into the nanofiltration unit is from 0.6 to 0.75. In other such embodiments, the weight ratio of the solvent to the HMO solution fed into the nanofiltration unit is no greater than 0.6. In other such embodiments, the weight ratio of the solvent to the HMO solution fed into the nanofiltration unit is from 0.09 to 0.6. In other such embodiments, the weight ratio of the solvent to the HMO solution fed into the nanofiltration unit is from 0.3 to 0.6. In other such embodiments, the weight ratio of the solvent to the HMO solution fed into the nanofiltration unit is less than 0.4. In other such embodiments, the weight ratio of the solvent to the HMO solution fed into the nanofiltration unit is from 0.09 to 0.4. In other such embodiments, the weight ratio of the solvent to the HMO solution fed into the nanofiltration unit is from 0.3 to 0.4. In other such embodiments, the weight ratio of the solvent to the HMO solution fed into the nanofiltration unit is less than 0.3. In other such embodiments, the weight ratio of the solvent to the HMO solution fed into the nanofiltration unit is from 0.09 to 0.3.

The above water-to-feed weight ratios are based on an HMO solution feed wherein the dry substance composition (*i.e.,* weight percent of the HMO solution feed that consists of non-water components) has been normalized to 4% (w/w). If the HMO solution being used has a different dry substance composition, the water-to-feed ratios can generally be converted proportionately. For example, a water-to-feed weight ratio of 0.6 for an HMO solution feed having a dry substance composition normalized to 4% (w/w) corresponds to a water-to-feed weight ratio of 0.3 for an HMO solution feed having a 2% (w/w) dry substance composition (*i.e.,* 0.3 = 0.6 x (2% ÷ 4%). Conversely, a water-to-feed weight ratio for an HMO solution feed having a specific dry substance composition can be converted into a water-to-feed weight ratio for an HMO solution feed having a dry substance composition normalized to 4% (w/w). To illustrate, if 1376 kg of HMO solution feed having a 2.9% (w/w) dry substance composition is fed into a nanofiltration unit along with 617 kg of diafiltration water, the water-to-feed weight ratio is 0.448 (this equals 617 ÷ 1376). This corresponds a water-to-feed weight ratio of 0.618 for an HMO solution feed having a dry substance composition normalized to 4% (*i.e.,* 0.618 = 0.448 x (4% ÷ 2.9%).

In some embodiments, the nanofiltration comprises diafiltration wherein water is fed into the nanofiltration unit with the HMO solution at a water-to-feed weight ratio of less than about 1.6 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of no greater than 1.5 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of no greater than 1.0 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of from 0.6 to 0.9 (normalized to an HMO solution having a dry substance composition of 4% (w/w)). In some such embodiments, the nanofiltration provides:

- a salt removal of at least about 40%, and/or
- a yield at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of from 0.6 to 0.75 (normalized to an HMO solution having a dry substance composition of 4% (w/w)). In some such embodiments, the nanofiltration provides:

- a salt removal of at least about 40%, and/or
- a yield of at least 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of from 0.3 to 0.6 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of less than 0.4 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of no greater than 0.3 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40% to about 96%. In other such embodiments, the salt removal is from 40% to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0% to 99.9%. In other such embodiments, the yield is at least 90.0%. In other such embodiments, the yield is from 90.0% to 99.9%. In other such embodiments, the yield is greater than 90.0%. Other such embodiments include any combination of the foregoing salt removals and yields.

In some embodiments, the nanofiltration comprises diafiltration wherein water is fed into the nanofiltration unit with the HMO solution at a water-to-feed weight ratio of from 0.09 to 0.3 (normalized to an HMO solution having a dry substance composition of 4% (w/w)). In some such embodiments, the nanofiltration provides:

- a salt removal of at least about 40%, and/or
- a yield of at least about 85.0% for the desired HMO.

In some such embodiments, the salt removal is from about 40 to about 96%. In other such embodiments, the salt removal is from 40 to 96%. In other such embodiments, the salt removal is at least 68%. In other such embodiments, the salt removal is from 68 to 96%.In other such embodiments, the salt removal is at least 90%. In other such embodiments, the salt removal is from 90 to 96%. In some such embodiments, the yield of the desired HMO is from 85.0 to 99.9%. In other such embodiments, the yield is at least 90.0%. In other such embodiments, the yield is from 90.0 to 99.9%. In other such embodiments, the yield is greater than 90.0%. Other such embodiments include any combination of the foregoing salt removals and yields.

In some embodiments, salt(s) removed from the HMO solution comprise(s) NaCl, $MgSO_4$, potassium phosphates (such as $KH_2PO_4$ and $K_2HPO_4$), and/or ammonium salts (such as ammonium sulfates and ammonium phosphates).

In some embodiments, salt(s) removed from the HMO solution comprise(s) NaCl and/or $MgSO_4$.

In some embodiments, the nanofiltration unit comprises a membrane having an $MgSO_4$ retention of at least about 50% when measured at a temperature of 25°C, concentration of 2 g/l, pressure of 8 bar, and pH of from 6 to 7. In some such embodiments, the $MgSO_4$ retention is from about 50 to about 99%. In other such embodiments, the $MgSO_4$ retention is from 50 to 99%. In other such embodiments, the $MgSO_4$ retention is from 70 to 98%. In other such embodiments, the $MgSO_4$ retention is from 75 to 98%. In other such embodiments, the $MgSO_4$ retention is from 80 to 97%. In other such embodiments, the $MgSO_4$ retention is from 90 to 97%. In other such embodiments, the $MgSO_4$ retention is from 90 to 96%. In other such embodiments, the $MgSO_4$ retention is greater than 90%. In other such embodiments, the $MgSO_4$ retention is greater than 90% and no greater than about 98%. In other such embodiments, the $MgSO_4$ retention is at least 90.5%. In other such embodiments, the $MgSO_4$ retention is from 90.5 to 98%. In other such embodiments, the $MgSO_4$ retention is at least 91%. In other such embodiments, the $MgSO_4$ retention is from 91 to about 98%. In other such embodiments, the $MgSO_4$ retention is from 91 to 96%. In other such embodiments, the $MgSO_4$ retention is at least 92%. In other such embodiments, the $MgSO_4$ retention is from 92 to about 98%. In other such embodiments, the $MgSO_4$ retention is from 92 to 96%.

In some embodiments, the nanofiltration unit comprises a membrane having an NaCl retention of up to about 60% (*i.e.,* from 0 to about 60%) when measured at a temperature of 25°C, concentration of 2 g/l, pressure of 8 bar, and pH of from 6 to 7. In some such embodiments, the NaCl retention is from 5 to 40%. In other such embodiments, the NaCl retention is from 10 to 35%. In other such embodiments, the NaCl retention is from 10 to 25%. In other such embodiments, the NaCl retention is from 14 to 22%. In other such embodiments, the NaCl retention is from 16 to 20%.

In some embodiments, the nanofiltration unit comprises a membrane having both an $MgSO_4$ retention of at

least about 50% and an NaCl retention of up to about 60% when measured at a temperature of 25°C, concentration of 2 g/l, pressure of 8 bar, and pH of from 6 to 7. In some such embodiments, the $MgSO_4$ retention is from about 50 to about 99% and the NaCl retention is up to about 60%. In other such embodiments, the $MgSO_4$ retention is from 80 to 97% and the NaCl retention is from 10 to 25%.

In some embodiments, the nanofiltration unit comprises a membrane having an $SO_4$ retention of at least about 50% when measured at a temperature of from 5 to 15°C, concentration of from 2,000 to 15,000 mg/kg, a flux of from 5 to 20 kg/m$^2$/hr, and pH of from 6 to 7. In some such embodiments, the $SO_4$ retention is from about 50% to about 99%. In other such embodiments, the $SO_4$ retention is from 70% to 98%. In other such embodiments, the $SO_4$ retention is from 80% to 98%. In other such embodiments, the $SO_4$ retention is from 90% to 97%. In other such embodiments, the $SO_4$ retention is from 91% to 96%. In other such embodiments, the $SO_4$ retention is from 94% to 96%.

In some embodiments, the nanofiltration unit comprises a membrane having a $PO_4$ retention of up to about 98% (*i.e.,* from 0 to about 98%) when measured at a temperature of from 5 to 15°C, concentration of from 2,000 to 15,000 mg/kg, a flux of from 5 to 20 kg/m$^2$/hr, and pH of from 6 to 7. In some such embodiments, the $PO_4$ retention is from about 30 to about 90%. In some such embodiments, the $PO_4$ retention is up to 70%. In other such embodiments, the $PO_4$ retention is from 40 to 60%. In other such embodiments, the $PO_4$ retention is from 50 to 60%. In other such embodiments, the $PO_4$ retention is from 52 to 55%.

In some embodiments, the nanofiltration unit comprises a membrane having a lactose retention of at least about 80% when measured at a temperature of 25°C, a concentration of 20 g/L, a flux of from 5 to 20 kg/m$^2$/hr, and pH of 8. In some such embodiments, the lactose retention is from about 80 to about 99%. In other such embodiments, the lactose retention is from 80 to 99%. In other such embodiments, the lactose retention is from 80 to 99%. In other such embodiments, the lactose retention is from 85 to 98%. In other such embodiments, the lactose retention is from 89 to 97%. In other such embodiments, the lactose retention is from 89 to 96%. In other such embodiments, the lactose retention is from 89 to 93%. In other such embodiments, the lactose retention is greater than 90%. In other such embodiments, the lactose retention is from 90.5 to about 99%. In other such embodiments, the lactose retention is at least 91%. In other such embodiments, the lactose retention is about 91%. In other such embodiments, the lactose retention is from 91 to about 99%.

In some embodiments, the nanofiltration membrane has a molecular weight cut-off of at least about 300 Dalton. In some embodiments, the nanofiltration has a molecular weight cut-off of no greater than about 1000 Dalton. In some embodiments, the nanofiltration membrane has a molecular weight cut-off of from about 300 to about 1000 Dalton. In some embodiments, the nanofiltration membrane has a molecular weight cut-off of from 300 to 800 Dalton. In some embodiments, the nanofiltration membrane has a molecular weight cut-off of from 300 to 750 Dalton. In some embodiments, the nanofiltration membrane has a molecular weight cut-off of from 300 to 700 Dalton. In some embodiments, the nanofiltration membrane has a molecular weight cut-off of from 300 to 650 Dalton. In some embodiments, the nanofiltration membrane has a molecular weight cut-off of from 300 to 600 Dalton. In some embodiments, the nanofiltration membrane has a molecular weight cut-off of less than 600 Dalton. In some embodiments, the nanofiltration membrane has a molecular weight cut-off of no greater than 590 Dalton. In some embodiments, the nanofiltration membrane has a molecular weight cut-off of from 300 to 590 Dalton. In some embodiments, the nanofiltration membrane has a molecular weight cut-off of less than 590 Dalton. In some embodiments, the nanofiltration membrane has a molecular weight cut-off of from 300 to 550 Dalton. In some embodiments, the nanofiltration membrane has a molecular weight cut-off of from about 300 to about 500 Dalton. In some embodiments, the nanofiltration membrane has a molecular weight cut-off of no greater than 500 Dalton. In some embodiments, the nanofiltration membrane has a molecular weight cut-off of from 300 to 500 Dalton. In some embodiments, the nanofiltration membrane has a molecular weight cut-off of from 350 to 500 Dalton. In some embodiments, the nanofiltration membrane has a molecular weight cut-off of less than 500 Dalton. In some embodiments, the nanofiltration membrane has a molecular weight cut-off of from 350 to 450 Dalton. In some embodiments, the nanofiltration membrane has a molecular weight cut-off of from 400 to 450 Dalton.

In some embodiments, the nanofiltration unit comprises a membrane having a molecular weight cut-off of from about 300 to about 800 Dalton and an $MgSO_4$ retention of at least about 50% when measured at a temperature of 25°C, concentration of 2 g/l, pressure of 8 bar, and pH of from 6 to 7. In some such embodiments, the molecular weight cut-off is from about 300 to about 750 Dalton and an $MgSO_4$ retention is from 50 to 99%. In other such embodiments, the molecular weight cut-off is from about 300 to about 700 Dalton and an $MgSO_4$ retention is from 50 to 99%. In other such embodiments, the molecular weight cut-off is from about 300 to about 650 Dalton and an $MgSO_4$ retention is from 50 to 99%. In other such embodiments, the molecular weight cut-off is from about 300 to about 600 Dalton and an $MgSO_4$ retention is from 50 to 99%. In other such embodiments, the molecular weight cut-off is less than 600 Dalton and an $MgSO_4$ retention is from 50 to 99%. In other such embodiments, the molecular weight cut-off is from about 300 to about 500 Dalton and an $MgSO_4$ retention is from 50 to 99%. In other such embodiments, the molecular weight cut-off is from about 300 to about 500 Dalton and an $MgSO_4$ retention is from 70 to 98%. In other such embodiments, the molecular weight cut-off is from about 300 to about 500 Dalton and an $MgSO_4$ retention is from 80 to 97%. In other such embodiments, the molecular weight cut-off is from about 300 to about 500 Dalton and an $MgSO_4$ retention is from 90 to 96%.

In some embodiments, the membrane is TRISEP® XN45 (Microdyn-Nadir (Goleta, CA)). XN45 reportedly has an approximate molecular weight cut-off of 300-500 Dalton; and 96% $MgSO_4$ retention at a temperature of 25°C, a concentration of 2 g/l concentration, a pressure of from 7.6 bar, a temperature of 25°C, and a pH of 8.

In some embodiments, the nanofiltration is carried out at a temperature of no greater than about 18°C. In some embodiments, the nanofiltration is carried out at a temperature of no greater than about 18°C. In some embodiments, the nanofiltration is carried out at a temperature of from 2 to 18°C. In some embodiments, the nanofiltration is carried out at a temperature of less than 18°C. In some embodiments, the nanofiltration is carried out at a temperature of no greater than 16°C. In some embodiments, the nanofiltration is carried out at a temperature of from 2 to 16°C. In some embodiments, the nanofiltration is carried out at a temperature of from 4 to 16°C. In some embodiments, the nanofiltration is carried out at a temperature of no greater than 15°C. In some embodiments, the nanofiltration is carried out at a temperature of from 4 to 15°C. In some embodiments, the nanofiltration is carried out at a temperature of from 5 to 15°C. In some embodiments, the nanofiltration is carried out at a temperature of no greater than 14°C. In some embodiments, the nanofiltration is carried out at a temperature of from 5 to 14°C. In some embodiments, the nanofiltration is carried out at a temperature of no greater than 12°C. In some embodiments, the nanofiltration is carried out at a temperature of from 5 to 12°C. In some embodiments, the nanofiltration is carried out at a temperature of no greater than about 10°C. In some embodiments, the nanofiltration is carried out at a temperature of no greater than 10°C. In some embodiments, the nanofiltration is carried out at a temperature of less than 10°C. In some embodiments, the nanofiltration is carried out at a temperature of from 4 to 10°C. In some embodiments, the nanofiltration is carried out at a temperature of from 5 to 10°C. In some embodiments, the nanofiltration is carried out at a temperature of less than 10°C. In some embodiments, the nanofiltration is carried out at a temperature of from 2 to 9.5°C. In some embodiments, the nanofiltration is carried out at a temperature of from 3 to 9.5°C. In some embodiments, the nanofiltration is carried out at a temperature of less than 9.5°C. In some embodiments, the nanofiltration is carried out at a temperature of no greater than 9°C. In some embodiments, the nanofiltration is carried out at a temperature of from 2 to 9°C. In some embodiments, the nanofiltration is carried out at a temperature of from 4 to 9°C. In some embodiments, the nanofiltration is carried out at a temperature of from 5 to 9°C. In some embodiments, the nanofiltration is carried out at a temperature of less than 9°C. In some embodiments, the nanofiltration is carried out at a temperature of from 2 to 8°C. In some embodiments, the nanofiltration is carried out at a temperature of from 3 to 8°C. In some embodiments, the nanofiltration is carried out at a temperature of less than 8°C. In some embodiments, the nanofiltration is carried out at a temperature of from 2 to 7°C. In some embodiments, the nanofiltration is carried out at a temperature of from 3 to 7°C.

Carrying out nanofiltration at a temperature of no greater than 18°C can be advantageous to, for example, improve microbiological stability. The term "improve microbiological stability" as used here refers to less or no growth of microorganisms in the HMO solution and/or concentrate during nanofiltration compared to nanofiltration at a greater temperature (such as 20°C or greater), with all other factors being the same.

In some embodiments, there is low or no growth of microorganisms in the HMO solution and/or concentrate during nanofiltration of the HMO solution.

In some embodiments, the nanofiltration is carried out for at least about 1 day. In some embodiments, the nanofiltration is carried out for no greater than about 5 days. In some embodiments, the nanofiltration is carried out for from about 1 to about 5 days. In some embodiments, the nanofiltration is carried out for from 1 to 4 days. In some embodiments, the nanofiltration is carried out for from 1 to 3 days. In some embodiments, the nanofiltration is carried out for at least 1 hr. In some embodiments, the nanofiltration is carried out for no greater than 48 hr. In some embodiments, the nanofiltration is carried out for from 1 to 48 hr. In some embodiments, the nanofiltration is carried out for from 3 to 48 hr. In some embodiments, the nanofiltration is carried out for from 6 to 48 hr. In some embodiments, the nanofiltration is carried out for from 9 to 48 hr. In some embodiments, the nanofiltration is carried out for no greater than 24 hr. In some embodiments, the nanofiltration is carried out for from 1 to 24 hr. In some embodiments, the nanofiltration is carried out for from 3 to 24 hr. In some embodiments, the nanofiltration is carried out for from 6 to 24 hr. In some embodiments, the nanofiltration is carried out for from 9 to 24 hr. In some embodiments, the nanofiltration is carried out for no greater than 18 hr. In some embodiments, the nanofiltration is carried out for from 1 to 18 hr. In some embodiments, the nanofiltration is carried out for from 3 to 18 hr. In some embodiments, the nanofiltration is carried out for from 6 to 18 hr. In some embodiments, the nanofiltration is carried out for from 9 to 18 hr. In some embodiments, the nanofiltration is carried out for no greater than 15 hr. In some embodiments, the nanofiltration is carried out for from 1 to 15 hr. In some embodiments, the nanofiltration is carried out for 3 to 15 hr. In some embodiments, the nanofiltration is carried out for from 6 to 15 hr. In some embodiments, the nanofiltration is carried out for from 9 to 15 hr. In some embodiments, the nanofiltration is carried out for no greater than 12 hr. In some embodiments, the nanofiltration is carried out for from 1 to 12 hr. In some embodiments, the nanofiltration is carried out for from 3 to 12 hr. In some embodiments, the nanofiltration is carried out for from 6 to 12 hr. In some embodiments, the nanofiltration is carried out for no greater than 9 hr. In some embodiments, the nanofiltration is carried out for from 9 to 12 hr. In some embodiments, the nanofiltration is carried out for from 1 to 9 hr. In some embodiments, the nanofiltration is carried out for from 3 to 9 hr. In some embodiments, the nanofiltration is carried out for from 6 to 9 hr. In some embodiments, the nanofiltration is carried out for about 9 hr. In some embodiments, the nanofiltration is carried out for about 12 hr.

In some embodiments, the nanofiltration is carried out at a flux of from 2 to 20 kg/$m^2$/hr (or L/$m^2$/hr (also referred to as "LMH")). In some embodiments, the nanofiltration is carried out at a flux of from 5 to 20 kg/$m^2$/hr (or L/$m^2$/hr). In some embodiments, the nanofiltration is carried out at a flux of from 10 to 20 kg/$m^2$/hr (or L/$m^2$/hr). Here, the term "flux" refers to the average flux throughout the nanofiltration.

In some embodiments, the nanofiltration is carried out using an HMO solution having a concentration of the desired HMO of from about 40 to about 200 g/L. In some embodiments, the nanofiltration is carried out using an HMO solution having a concentration of the desired HMO of from 50 to 150 g/L.

In some embodiments, the nanofiltration is carried out at a pH of less than about 6.4. In some embodiments, the nanofiltration is carried out at a pH of greater than about 3.0. In some embodiments, the nanofiltration is carried out at a pH of from about 3.0 to about 6.4 In some embodiments, the nanofiltration is carried out at a pH of less than 6.0. In some embodiments, the nanofiltration is carried out at a pH of from 3.0 to 5.5. In some embodiments, the nanofiltration is carried out at a pH about 5.5. In some embodiments, the nanofiltration is carried out at a pH of less than 5.5. In some embodiments, the nanofiltration is carried out at a pH about 5.4. In some embodiments, the nanofiltration is carried out at a pH of less than 5.4. In some embodiments, the nanofiltration is carried out at a pH of from 3.0 to 5.3. In some embodiments, the nanofiltration is carried out at a pH of from 3.2 to 5.3. In some embodiments, the nanofiltration is carried out at a pH about 5.3. In some embodiments, the nanofiltration is carried out at a pH of less than 5.3. In some embodiments, the nanofiltration is carried out at a pH of from 3.2 to 5.2. In some embodiments, the nanofiltration is carried out at a pH of from 3.3 to 5.2. In some embodiments, the nanofiltration is carried out at a pH of from 3.4 to 5.2. In some embodiments, the nanofiltration is carried out at a pH about 5.2. In some embodiments, the nanofiltration is carried out at a pH of less than 5.2. In some embodiments, the nanofiltration is carried out at a pH about 5.1. In some embodiments, the nanofiltration is carried out at a pH about 5.0. In some embodiments, the nanofiltration is carried out at a pH of less than 5.0. In some embodiments, the nanofiltration is carried out at a pH of less than 4.5. In some embodiments, the nanofiltration is carried out at a pH of less than 4.0. In some embodiments, the nanofiltration is carried out at a pH about 3.5. In some embodiments, the nanofiltration is carried out at a pH of less than 3.5. In some embodiments, the nanofiltration is carried out at a pH about 3.4. In some embodiments, the nanofiltration is carried out at a pH less than 3.3.

In some embodiments, nanofiltration of an HMO solution is conducted at a low pH *(e.g.,* from about 3.0 to about 5.5) to improve the membrane's retention for the desired HMO. For example, in some embodiments, a retention of greater than 97% for the desired HMO is obtained by using a pH of from about 3.0 to about 5.5, wherein use of a pH of greater than 6.0 would have instead lead to a retention of less than 97.0%.

**Additional treatments**

In some embodiments, the process comprises subjecting the HMO feed solution and/or nanofiltration concentrate to one or more of the following treatments: an enzymatic treatment (e.g., enzymatic hydrolysis of lactose), antifoam removal, electrodialysis, chromatography, cation exchange, anion exchange, mixed bed ion exchange, evaporation, activated carbon, crystallization, evaporation and/or spray-drying.

The additional treatments may typically be carried out in various orders, as well as being repeated at different points in the process. In some embodiments, the process comprises nanofiltration and a combination of at least two of the above additional treatments. In some embodiments, the process comprises nanofiltration and a combination of at least three of the above additional treatments.

In some embodiments, the process comprises an activated carbon treatment. An activated carbon treatment may be used to remove color-producing material and other undesirable material, such as larger oligosaccharides. In some embodiments, the process comprises two or more activated carbon treatments. In some embodiments, the process comprises an activated carbon treatment before a nanofiltration step. In some embodiments, the process comprises an activated carbon treatment after a nanofiltration step.

In some embodiments, the process comprises removal of antifoam used upstream (e.g., during the fermentation). In some embodiments, the process comprises an antifoam removal step before the nanofiltration.

In some embodiments, the process comprises at least one cation exchange step. In some such embodiments, the process comprises both a cation exchange step that occurs before a nanofiltration step. In other such embodiments, the process comprises a cation exchange step that occurs after a nanofiltration step. In some embodiments, the process comprises at least one anion exchange step. In some such embodiments, the process comprises both an anion exchange step that occurs before a nanofiltration step. In other such embodiments, the process comprises an anion exchange step that occurs after a nanofiltration step. In some embodiments, the process comprises both a cation exchange step and an anion exchange step. In some such embodiments, the process comprises both a cation exchange step and an anion exchange step that occur before a nanofiltration step. In some such embodiments, the process comprises both a cation exchange step and an anion exchange step that occur after a nanofiltration step.

In some embodiments, the process comprises (two or more) nanofiltration steps. A further nanofiltration can be helpful, for example, to further reduce the volume of a concentrate.

In some embodiments, the concentrate is subjected to evaporation. This can be helpful, for example, to concentrate the HMO by removing a solvent (e.g., water). In some embodiments, the evaporation is carried out at a temperature of from about 20 to about 80°C. In some embodiments, the evaporation is carried out at a temperature of from 25 to 75°C. In some embodiments, the evaporation is carried out at a temperature of from 30 to 70°C. In some embodiments, the evaporation is carried out at a temperature of from 30 to 65°C. In some embodiments, evaporation is the final purification step of the desired HMO.

In some embodiments, the concentrate is subjected to spray drying. In some embodiments, the HMO feed into the spray dryer has a Brix value of from about 8 to about 75%Brix. In some embodiments, the Brix value is from about 30 to about 65%Brix. In some embodiments, the Brix value is from about 50 to about 60%Brix. In some embodiments, the feed into the spray dryer is at a temperature of from about 2 to about 70°C immediately before being dispersed into droplets in the spray dryer. In some embodiments, the feed into the spray dryer is at a temperature of from about 30 to about 60°C immediately before being dispersed into droplets in the spray dryer. In some embodiments, the feed into the spray dryer is at a temperature of from about 2 to about 30°C immediately before being dispersed into droplets in the spray dryer. In some embodiments, the spray drying uses air having an air inlet temperature of from 120 to 280°C. In some embodiments, the air inlet temperature is from 120 to 210°C. In some embodiments, the air inlet temperature is from about 130 to about 190°C. In some embodiments, the air inlet temperature is from about 135 to about 160°C. In some embodiments, the spray drying uses air having an air outlet temperature of from about 80 to about 110°C. In some embodiments, the air outlet temperature is from about 100 to about 110°C. In some embodiments, the spray drying is carried out at a temperature of from about 20 to about 90°C. In some embodiments, the spray dryer is a co-current spray dryer. In some embodiments, the spray dryer is attached to an external fluid bed. In some embodiments, the spray dryer comprises a rotary disk, a high pressure nozzle or a two-fluid nozzle. In some embodiments, the spray dryer comprises an atomizer wheel. In some embodiments, spray-drying is the final purification step for the desired HMO.

In some embodiments, the process comprises crystallization. In some embodiments, no organic solvent is used during the crystallization. In some embodiments, the crystallization comprises a crystallization process disclosed in WO2018/164937 (incorporated by reference into this specification). In some embodiments, crystallization is the final purification step of the desired HMO. In some embodiments, the process comprises both crystallization and evaporation. In some embodiments, the process comprises both crystallization and spray-drying.

**Products Comprising HMOs**

In some embodiments, an HMO purified by a process of this specification is incorporated into a food (e.g., human or pet food), dietary supplement or medicine. In some embodiments, the HMO is mixed with one or more ingredients suitable as for a food, dietary supplement or medicine.

In some embodiments, the dietary supplement comprises at least one prebiotic ingredient and/or at least one probiotic ingredient.

A "prebiotic" is a substance that promotes growth of microorganisms beneficial to the host, particularly microorganisms in the gastrointestinal tract. In some embodiments, a dietary supplement provides multiple prebiotics, including at least one HMO purified by a process disclosed in this specification, to promote growth of one or more beneficial microorganisms. Examples of prebiotic ingredients for dietary supplements include other prebiotic molecules (such as other HMOs) and plant polysaccharides (such as inulin, pectin, β-glucan and xylooligosaccharide).

A "probiotic" product typically contains live microorganisms that replace or add to gastrointestinal microflora, to the benefit of the recipient. Examples of such microorganisms include *Lactobacillus* species (for example, *L. acidophilus* and *L. bulgaricus*), *Bifidobacterium* species (for example, *B. animalis, B. longum* and *B. infantis* (*e.g.,* Bi-26)), and *Saccharomyces boulardii.* In some embodiments, an HMO purified by a process of this specification is orally administered in combination with Bi-26.

Examples of further ingredients for dietary supplements include disaccharides (such as lactose), monosaccharides (such as glucose and galactose), thickeners (such as gum arabic), acidity regulators (such as trisodium citrate), water, skimmed milk, and flavourings.

In some embodiments, the HMO is incorporated into a human baby food (*e.g.,* infant formula). Infant formula is generally a manufactured food for feeding to infants as a complete or partial substitute for human breast milk. In some embodiments, infant formula is sold as a powder and prepared for bottle- or cup-feeding to an infant by mixing with water. The composition of infant formula is typically designed to be roughly mimic human breast milk. In some embodiments, an HMO purified by a process in this specification is included in infant formula to provide nutritional benefits similar to those provided by one or more HMOs in human breast milk. In some embodiments, the HMO is mixed with one or more ingredients of the infant formula. Examples of infant formula ingredients include nonfat milk, carbohydrate sources (*e.g.,* lactose), protein sources (*e.g.,* whey protein concentrate and casein), fat sources (*e.g.*, vegetable oils - such as palm, high oleic safflower oil, rapeseed, coconut and/or sunflower oil; and fish oils), vitamins (such as vitamins A, $B_6$, $B_{12}$, C and D), minerals (such as potassium citrate, calcium citrate, magnesium chloride, sodium chloride, sodium citrate and calcium phosphate) and other HMOs. Other HMOs may include, for example, DiFL, lacto-*N*-triose II, LNT, LNnT, lacto-*N*-fucopentaose I, lacto-*N-neo*fucopentaose, lacto-*N*-fucopentaose II, lacto-*N*-fucopentaose III, lacto-*N*-fucopentaose V, lacto-*N-neo*fucopentaose V, lacto-*N*-difucohexaose I, lacto-*N*-difucohexaose II, 6'-galactosyllactose, 3'-galactosyllactose, lacto-*N*-hexaose and lacto-*N-neo*hexaose.

In some embodiments, the one or more infant formula ingredients comprise nonfat milk, a carbohydrate source, a protein source, a fat source, and/or a vitamin and mineral.

In some embodiments, the one or more infant formula ingredients comprise lactose, whey protein concentrate and/or high oleic safflower oil.

In some embodiments, the HMO is dried HMO.

In some embodiments, the HMO concentration in the infant formula is approximately the same concentration as the HMO concentration generally present in human breast milk. In some embodiments, the concentration of each HMO in the infant formula is approximately the same concentration as the concentration of that HMO generally present in human breast milk.

EXAMPLES

The following examples are merely illustrative, and not limiting to this specification in any way.

**Example 1**

**Performance of Different Membranes at a Cold Temperature**

The process equipment included a plate and frame filtration unit (Alfa Laval Labstak M20), feed and diafiltration pump, heat exchanger, cooling unit, 70-liter feed tank as well as inlet and outlet pressure gauges and a pressure control valve. The total membrane area was 0.65 $m^2$. The tested membranes were TRISEP® UA60 (Microdyn-Nadir, approximate molecular weight cut-off of 600-1000 Dalton, 80% $MgSO_4$ retention at 25°C), TRISEP® XN45 (Microdyn-Nadir, approximate molecular weight cut-off of 300-500 Dalton, 90-96% $MgSO_4$ retention at 25°C), NF245 (Dow Chemical Company (Midland, MI), approximate molecular weight cut-off of 150-300 Dalton, >98% $MgSO_4$ retention at 25°C), DL (Suez Water Technologies & Solutions (Trevose, PA), approximate molecular weight cut-off of 150-300 Dalton, >98% $MgSO_4$ retention at 25°C) and DK (Suez, approximate molecular weight cut-off of 150-300 Dalton, >98% $MgSO_4$ retention at 25°C). $MgSO_4$ retention is specified at a 1-2 g/L concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

43 kg of feed was fed into the 70-liter feed tank. The dry substance concentration in the feed was 4.6 g/100 g, the conductivity was 11.4 mS/cm and the pH was 6.7. Permeate was collected from all the membranes. The feed composition is shown in **Table 1-1,** wherein the HPLC analyses are given on a percent dry substance basis.

**TABLE 1-1**

| Feed Composition (%) | |
|---|---|
| L-fucose | 0.0 |
| galactose + glucose | 1.5 |
| lactose | 1.1 |
| 3-fucosyllactose | 0.5 |
| 2' fucosyllactose | 31.3 |
| difucosyllactose | 2.1 |
| other | 63.5 |

The feed was kept cool at 10-14°C, and water was used for diafiltration. The filtration pressure was 10-30 bar, and the concentrate DS (dry substance) concentration was controlled to keep flux above 2 $kg/m^2/hr$ (the minimum flux point observed).

After batch filtration, one permeate fraction and a final concentrate fraction were collected. The results, including HPLC analyses on a percent dry substance basis for the permeate fraction and final concentrate, are shown in **Table 1-2.**

TABLE 1-2

| | Total permeate | Final concentrate |
|---|---|---|
| mass, kg | 38.5 | 4.7 |
| dry substance, g/100 g | 2.5 | 22.6 |
| conductivity mS/cm | 9.1 | 25.3 |
| L-fucose, % | 0.1 | 0.0 |
| galactose + glucose, % | 2.7 | 0.6 |
| lactose, % | 0.9 | 0.7 |
| 3-fucosyllactose, % | 0.3 | 0.5 |
| 2'fucosyllactose, % | 19.7 | 41.1 |
| difucosyllactose, % | 0.7 | 3.4 |
| other, % | 75.6 | 53.7 |

The overall 2'-FL yield calculated was 69.0%. The salt removal calculated from conductivity was 71.5%.

Sulfate removal was 45.3%, and phosphate removal was 63.0%. 2'-FL retention was measured from 3 points, and the average values were 88.1%, 99.3%, 99.4%, 98.3% and 97.9% for the UA60, XN45, NF245, DL and DK membranes, respectively. The conductivity retention was measured from 3 points, and the average values were 37.1%, 69.4%, 75.2%, 78.5% and 78.4% for UA60, XN45, NF245, DL and DK membranes, respectively.

$SO_4$, $PO_4$ and lactose retentions were calculated from results of the last sample set during the diafiltration where concentrations were the highest. The total dry substance concentration was 22.7 g/100 g and the concentration of 2'-FL was 9.28% (w/w), lactose 0.16% (w/w), $SO_4$ was 13660 mg/kg and $PO_4$ was 5020 mg/kg. $SO_4$ retentions were 49%, 92%, 97%, 95% and 94% for the UA60, XN45, NF245, DL and DK membranes, respectively. $PO_4$ retentions were 18%, 55%, 72%, 81% and 81% for the UA60, XN45, NF245, DL and DK membranes, respectively. Lactose retentions were 45%, 91%, 99%, 96% and 95% for the UA60, XN45, NF245, DL and DK membranes, respectively.

**Example 2**

**Performance of Different Membranes at an Elevated Temperature**

The process equipment included a plate and frame filtration unit (Alfa Laval Labstak M20), feed and diafiltration pump, heat exchanger, cooling unit, 70-liter feed tank as well as inlet and outlet pressure gauges and a pressure control valve. The total membrane area was 0.65 $m^2$. The tested membranes were TRISEP® UA60 (Microdyn-Nadir, approximate molecular weight cut-off of 600-1000 Dalton, 80% $MgSO_4$ retention at 25°C), TRISEP® XN45 (Microdyn-Nadir, approximate molecular weight cut-off of 300-500 Dalton, 90-96% $MgSO_4$ retention at 25°C), NF245 (DOW, approximate molecular weight cut-off of 150-300 Dalton, >98% $MgSO_4$ retention at 25°C), DL (Suez, approximate molecular weight cut-off of 150-300 Dalton, >98% $MgSO_4$ retention at 25°C) and DK (Suez, approximate molecular weight cut-off of 150-300 Dalton, >98% $MgSO_4$ retention at 25°C). $MgSO_4$ retention is specified at 1-2 g/l concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

53.5 kg of feed was fed into the 70-liter feed tank. The dry substance concentration of the feed was 4.2 g/100 g, the conductivity was 11.1 mS/cm and the pH was 6.9. Permeate was collected from all the membranes. The feed composition is shown in **Table 2-1,** wherein the HPLC analyses are given on a percent dry substance basis.

TABLE 2-1

| Feed Composition (%) | |
|---|---|
| L-fucose | 0.0 |
| galactose + glucose | 1.6 |
| lactose | 1.0 |

(continued)

| Feed Composition (%) | |
|---|---|
| 3-fucosyllactose | 0.5 |
| 2' fucosyllactose | 32.7 |
| difucosyllactose | 2.0 |
| other | 62.1 |

The feed was heated and kept at 50°C, and water was used for diafiltration. The filtration pressure was 6-40 bar, and the concentrate DS concentration was controlled to keep flux above 3 kg/$m^2$/hr (the minimum flux point observed).

After batch filtration, two permeate fractions and a final concentrate fraction were collected. The results, including HPLC analyses on a percent dry substance basis for the permeate fraction and final concentrate, are shown in **Table 2-2.**

**TABLE 2-2**

| | Total permeate 1 | Total permeate 2 | Final concentrate |
|---|---|---|---|
| mass, kg | 64.5 | 11.8 | 4.5 |
| dry substance, g/100 g | 2.1 | 0.8 | 20.2 |
| conductivity mS/cm | 7.9 | 2.4 | 14.0 |
| L-fucose, % | 0.0 | 0.1 | 0.0 |
| galactose + glucose, % | 3.1 | 0.8 | 0.1 |
| lactose, % | 1.0 | 1.1 | 0.9 |
| 3-fucosyllactose, % | 0.3 | 0.3 | 1.0 |
| 2'fucosyllactose, % | 18.8 | 23.7 | 50.8 |
| difucosyllactose, % | 0.6 | 0.8 | 4.3 |
| other, % | 76.1 | 73.2 | 42.9 |

The overall 2'-FL yield calculated was 63.4% and salt removal calculated from conductivity was 85.9%. Sulfate removal was 55.3%, and phosphate removal was 93.3%. 2'-FL retention was measured from 3 points, and the average values were 78.1%, 95.7%, 99.7%, 98.7% and 99.0% for the UA60, XN45, NF245, DL and DK membranes, respectively. The conductivity retention was measured from 3 points, and the average values were 38.8%, 57.0%, 70.5%, 75.2% and 76.4% for the UA60, XN45, NF245, DL and DK membranes, respectively.

$SO_4$, $PO_4$ and lactose retentions were calculated from the sample set where concentrations were the highest. The total dry substance concentration was 24.6 g/100 g and the concentration of 2'-FL was 10.31% (w/w), lactose was 0.23% (w/w), $SO_4$ was 13160 mg/kg and $PO_4$ was 6400 mg/kg. $SO_4$ retentions were 46%, 83%, 97%, 98% and 98% for the UA60, XN45, NF245, DL and DK membranes, respectively. $PO_4$ retentions were 16%, 18%, 38%, 69% and 50% for the UA60, XN45, NF245, DL and DK membranes, respectively. Lactose retentions were 63%, 71%, 99%, 94% and 97% for the UA60, XN45, NF245, DL and DK membranes, respectively.

**Example 3**

**Cold Temperature Concentration and Salt Removal of 2'-FL Liquid with NF Membranes**

The process equipment included a spiral wound membrane unit with two single membrane housings (GEA, Model R), feed and diafiltration pump, heat exchanger, cooling unit, 500-liter feed tank as well as inlet and outlet pressure gauges and a pressure control valve. The total membrane area was 13.4 $m^2$. The tested membranes were TRISEP® XN45 (Microdyn-Nadir, approximate molecular weight cut-off of 300-500 Dalton, 90-96% $MgSO_4$ retention at 25°C, 7.4 $m^2$ / 31 mil spacer size) and DL (Suez, approximate molecular weight cut-off of 150-300 Dalton, >98% $MgSO_4$ retention at 25°C, 6.0 $m^2$ / 50 mil spacer size). The membranes were installed in parallel. $MgSO_4$ retention is specified at 1-2 g/l concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

313 kg of feed was fed into a 500-liter feed tank. The dry substance concentration in the feed was 6.1 g/100 g, the conductivity was 14.0 mS/cm and the pH was 6.7. Permeate was collected from both membranes. The feed composition is shown in **Table 3-1,** wherein the HPLC analyses are given on a percent dry substance basis.

**TABLE 3-1**

| Feed Composition (%) | |
|---|---|
| L-fucose | <0.1 |
| galactose + glucose | <0.1 |
| lactose | 0.1 |
| 3-fucosyllactose | 0.4 |
| 2'fucosyllactose | 44.5 |
| difucosyllactose | 2.7 |
| other | 52.3 |

The feed was kept cool at 6-8°C, and water was used for diafiltration. The filtration pressure was 10-27 bar, and the concentrate DS concentration was controlled to keep fluxes above 2 kg/$m^2$/hr (the minimum flux point observed).

After batch filtration, two permeate fractions and a final concentrate fraction were collected. The results, including HPLC analyses on a percent dry substance basis for the permeate fraction and final concentrate, are shown in **Table 3-2.**

**TABLE 3-2**

| | Total permeate 1 | Total permeate 2 | Final concentrate |
|---|---|---|---|
| mass, kg | 193.0 | 158.8 | 68 |
| dry substance, g/100 g | 0.6 | 1.4 | 21.0 |
| conductivity mS/cm | 5.8 | 10.6 | 26.7 |
| L-fucose, % | 0.1 | <0.1 | <0.1 |
| galactose + glucose, % | 0.2 | 0.2 | <0.1 |
| lactose, % | 0.1 | 0.1 | 0.1 |
| 3-fucosyllactose, % | 0.0 | 0.3 | 0.4 |
| 2'fucosyllactose, % | 2.0 | 4.2 | 54.0 |
| difucosyllactose, % | 0.2 | 0.3 | 5.0 |
| other, % | 97.6 | 95.0 | 40.4 |

The overall 2'-FL yield calculated was 98.6% based on feed and permeate fractions, and salt removal calculated from conductivity was 58.6%. Sulfate removal was 24.4%, and phosphate removal was 62.3%. 106.5 kg of water was used, and, thus, the water-to-feed weight ratio normalized to a 4% (w/w) dry substance solution was 0.22 kg/kg (*i.e.,* the weight ratio of water to feed corresponds to 0.22 for a feed solution having a 4% (w/w) dry substance concentration). 2'-FL retention was measured from 4 points, and the average values were 99.6% and 98.9% for the XN45 and DL membranes, respectively. The conductivity retention was measured from 4 points, and the average values were for 66.6%, 78.6%, for XN45 and DL membranes, respectively. Permeability of the XN45 membrane was, on average, 2.2 greater than that of the DL membrane during the filtration.

**Example 4**

**Elevated Temperature Concentration and Salt Removal of 2'-FL Liquid with NF Membranes**

The process equipment included a spiral wound membrane unit with two single membrane housings (GEA, Model R), feed and diafiltration pump, heat exchanger, 500-liter feed tank as well as inlet and outlet pressure gauges and a pressure control valve. The total membrane area was 13.4 $m^2$. The tested membranes were TRISEP® XN45 (Microdyn-Nadir, approximate molecular weight cut-off of 300-500 Dalton, 90-96% $MgSO_4$ retention at 25°C, 7.4 $m^2$ / 31 mil spacer size) and DL (Suez, approximate molecular weight cut-off of 150-300 Dalton, >98% $MgSO_4$ retention at 25°C, 6.0 $m^2$ / 50 mil spacer size). The membranes were installed in parallel. $MgSO_4$ retention is specified at 1-2 g/l concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

285 kg of feed was fed into a 500-liter feed tank. The dry substance concentration in the feed was 7.4 g/100 g, the conductivity was 14.4 mS/cm and the pH was 6.7. Permeate was collected from both membranes. The feed composition is shown in **Table 4-1,** wherein the HPLC analyses are given on a percent dry substance basis.

**TABLE 4-1**

| Feed Composition (%) | |
|---|---|
| L-fucose | <0.1 |
| galactose + glucose | 0.2 |
| lactose | 0.1 |
| 3-fucosyllactose | 0.3 |
| 2'fucosyllactose | 42.9 |
| difucosyllactose | 2.6 |
| other | 54.1 |

The feed was heated and maintained at 58-61°C, and water was used for diafiltration. The filtration pressure was 10-25 bar, and the concentrate DS concentration was controlled to keep fluxes above 2 kg/$m^2$/hr (the minimum flux point observed).

After batch filtration, a permeate fraction and a final concentrate fraction were collected. The results, including HPLC analyses on a percent dry substance basis for the permeate fraction and final concentrate, are shown in **Table 4-2.**

**TABLE 4-2**

| | Total permeate | Final concentrate |
|---|---|---|
| mass, kg | 423.0 | 34.0 |
| dry substance, g/100 g | 2.8 | 27.1 |
| conductivity mS/cm | 12.1 | 8.8 |
| L-fucose, % | <0.1 | 0.0 |
| galactose + glucose, % | <0.1 | 0.0 |
| lactose, % | 0.6 | 0.0 |
| 3-fucosyllactose, % | 0.0 | 0.2 |
| 2'fucosyllactose, % | 35.5 | 60.8 |
| difucosyllactose, % | 0.3 | 7.6 |
| other, % | 63.6 | 31.4 |

The overall 2'-FL yield calculated was 54.3% based on feed and permeate fractions, and salt removal calculated from conductivity was 92.7%. Sulfate removal was 90.4%, and phosphate removal was 99.8%. 172.0 kg of water was used, and, thus, the water-to-feed weight ratio normalized to a 4% (w/w) dry substance solution was 0.33 kg/kg (*i.e.,* the weight ratio of water to feed corresponds to 0.33 for a feed solution having a 4% (w/w) dry substance concentration). 2'-FL retention was measured from 2 points, and the average values were 91.2% and 97.7% for the XN45 and DL membranes, respectively. The conductivity retention was measured from 2 points, and the average values were for 32.9%, 58.0%, for XN45 and DL membranes, respectively. Permeability of the XN45 membrane was, on average, 5.2 greater than that of the DL membrane during the filtration.

**Example 5**

**Elevated Temperature Concentration and Salt Removal of 2'-FL Liquid with Tight NF Membranes**

The process equipment included a spiral wound membrane unit with two single membrane housings (GEA, Model R), feed and diafiltration pump, heat exchanger, 500-liter feed tank as well as inlet and outlet pressure gauges and a pressure control valve. The total membrane area was 12.0 $m^2$. The tested membranes were DL (Suez, approximate molecular weight cut-off of 150-300 Dalton, >98% $MgSO_4$ retention at 25°C, 6.0 $m^2$ / 50 mil spacer size). The membranes were installed in parallel. $MgSO_4$ retention is specified at 1-2 g/l concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

162 kg of feed was fed into a 500-liter feed tank. The dry substance concentration in the feed was 7.3 g/100 g, the conductivity was 17.4 mS/cm and the pH was 6.9. Permeate was collected from both membranes. The feed composition is shown in **Table 5-1,** wherein the HPLC analyses are given on a percent dry substance basis.

**TABLE 5-1**

| Feed Composition (%) | |
|---|---|
| L-fucose | <0.1 |
| galactose + glucose | 0.2 |
| lactose | 0.1 |
| 3-fucosyllactose | 0.3 |
| 2'fucosyllactose | 42.9 |
| difucosyllactose | 2.6 |
| other | 54.1 |

The feed was heated and kept at 58-61°C, and water was used for diafiltration. The filtration pressure was 10-25 bar, and the concentrate DS concentration was controlled to keep fluxes above 2 kg/$m^2$/hr (the minimum flux point observed).

After batch filtration, a permeate fraction and a final concentrate fraction were collected. The results, including HPLC analyses on a percent dry substance basis for the permeate fraction and final concentrate, are shown in **Table 5-2.**

**TABLE 5-2**

| | Total permeate | Final concentrate |
|---|---|---|
| mass, kg | 275.0 | 35.0 |
| dry substance, g/100 g | 1.0 | 25.8 |
| conductivity mS/cm | 6.8 | 22.2 |
| L-fucose, % | 0.0 | 0.0 |
| galactose + glucose, % | 0.0 | 0.0 |
| lactose, % | 0.7 | 0.0 |
| 3-fucosyllactose, % | 0.0 | 0.0 |
| 2'fucosyllactose, % | 4.5 | 61.7 |
| difucosyllactose, % | 0.2 | 5.3 |
| other, % | 94.6 | 32.9 |

The overall 2'-FL yield calculated was 97.7% based on feed and permeate fractions, and salt removal calculated from conductivity was 72.4%. Sulfate removal was 43.4%, and phosphate removal was 93.6%. 148.0 kg of water was used, and, thus, the water-to-feed weight ratio normalized to a 4% (w/w) dry substance solution was 0.50 kg/kg (*i.e.,*

the weight ratio of water to feed corresponds to 0.50 for a feed solution having a 4% (w/w) dry substance concentration). 2'-FL retention was measured from 2 points, and the average value was 99.3%. The conductivity retention was measured from 3 points, and the average value was 61.5%.

**Example 6**

**Cold Temperature Concentration and Salt Removal of 2'-FL Liquid with Open NF Membranes**

The process equipment included a spiral wound membrane unit with two single membrane housings (GEA, Model R), feed and diafiltration pump, heat exchanger, 500-liter feed tank as well as inlet and outlet pressure gauges and a pressure control valve. The total membrane area was 14.8 $m^2$. The tested membranes were TRISEP® XN45 (Microdyn-Nadir, approximate molecular weight cut-off of 300-500 Dalton, 90-96% $MgSO_4$ retention at 25°C, 7.4 $m^2$ / 31 mil spacer size). The membranes were installed in parallel. $MgSO_4$ retention is specified at 1-2 g/l concentration in 7-8 bar, 25°C, pH 6-8 and 10-25% recovery.

Ultrafiltration permeate from an *E. coli* based fermentation of 2'-FL was used as a feed. The aim was to concentrate 2'-FL contained in the feed while passing impurities (salts, monomeric sugars, dimeric sugars) to the permeate.

1200 kg of feed was fed into a 500-liter feed tank during concentration. The dry substance concentration in the feed was 3.8 g/100 g, the conductivity was 12.7 mS/cm and the pH was 6.9. Permeate was collected from both membranes. The feed composition is shown in **Table 6-1,** wherein the HPLC analyses are given on a percent dry substance basis.

**TABLE 6-1**

| Feed Composition (%) | |
|---|---|
| L-fucose | 0.0 |
| galactose + glucose | 0.0 |
| lactose | 3.5 |
| 3-fucosyllactose | 0.0 |
| 2'fucosyllactose | 44.9 |
| difucosyllactose | 3.0 |
| other | 48.6 |

The feed was kept cool at 4-7°C, and water was used for diafiltration. The filtration pressure was 10-23 bar, and the concentrate DS concentration was controlled to keep fluxes above 3 kg/$m^2$/hr (the minimum flux point observed).

After batch filtration, two permeate fractions and a final concentrate fraction were collected. The results, including HPLC analyses on a percent dry substance basis for the permeate fraction and final concentrate, are shown in **Table 6-2.**

**TABLE 6-2**

| | Total permeate 1 | Total permeate 2 | Final concentrate |
|---|---|---|---|
| mass, kg | 972.0 | 86.6 | 250.0 |
| dry substance, g/100 g | 0.6 | 1.0 | 16.8 |
| conductivity mS/cm | 4.8 | 8.2 | 34.3 |
| L-fucose, % | 0.0 | 0.0 | 0.0 |
| galactose + glucose, % | 0.0 | 0.0 | 0.0 |
| lactose, % | 1.9 | 4.4 | 3.7 |
| 3-fucosyllactose, % | 0.0 | 0.0 | 0.0 |
| 2'fucosyllactose, % | 4.5 | | 53.8 |
| difucosyllactose, % | 0.2 | | 4.3 |
| other, % | 94.6 | | 38.2 |

The overall 2'-FL yield calculated was 99.3% based on feed and permeate fractions, and salt removal calculated from conductivity was 43.9%. Sulfate removal was 29.1%, and phosphate removal was 46.2%. 108.6 kg of water was used, and, thus, the water-to-feed weight ratio normalized to a 4% (w/w) dry substance solution was 0.10 kg/kg (*i.e.,* the weight ratio of water to feed corresponds to 0.10 for a feed solution having a 4% (w/w) dry substance concentration). 2'-FL retention was measured from 2 points, and the average value was 99.8%. The conductivity retention was measured from 2 points, and the average value was 78.5%.

**Example 7**

**3-FL Liquid Concentration and Salt Removal in Cold Temperature Nanofiltration**

The process equipment included a spiral wound membrane unit with two single membrane housings (GEA, Model R), feed and diafiltration pump, heat exchanger, 500-liter feed tank as well as inlet and outlet pressure gauges and a pressure control valve. The total membrane area was 14.8 $m^2$. The tested membranes were TRISEP® XN45 (Microdyn-Nadir, approximate molecular weight cut-off of 300-500 Dalton, 90-96% $MgSO_4$ retention at 25°C, 7.4 $m^2$ / 31 mil spacer size). The membranes were installed in parallel. $MgSO_4$ retention is specified at 1-2 g/l concentration in 7-8 bar, 25°C, pH 6-8 and 10-25% recovery.

Ultrafiltration permeate from an *E. coli* based fermentation of 3-FL was used as a feed. The aim was to concentrate 3-FL contained in the feed while passing impurities (salts, monomeric sugars, dimeric sugars) to the permeate.

1376 kg of feed was fed into a 500-liter feed tank during concentration. The dry substance concentration in the feed was 2.9 g/100 g, the conductivity was 12.3 mS/cm and the pH was 7.6. Permeate was collected from both membranes. The feed composition is shown in Table 7-1, wherein the HPLC analyses are given on a percent dry substance basis.

**TABLE 7-1**

| Feed Composition (%) | |
|---|---|
| L-fucose | 0.0 |
| galactose + glucose | 0.0 |
| lactose | 1.6 |
| 3-fucosyllactose | 44.6 |
| 2'fucosyllactose | 0.0 |
| difucosyllactose | 0.0 |
| other, % | 53.8 |

The feed was kept cool at 3-7°C, and water was used for diafiltration. The filtration pressure was 10-30 bar, and the concentrate DS concentration was controlled to keep fluxes above 8 kg/$m^2$/hr (the minimum flux point observed).

After batch filtration, two permeate fractions and a final concentrate fraction were collected. The results, including HPLC analyses on a percent dry substance basis for the permeate fraction and final concentrate, are shown in **Table 7-2.**

**TABLE 7-2**

| | Total permeate 1 | Total permeate 2 | Final concentrate |
|---|---|---|---|
| mass, kg | 940.0 | 883.0 | 170.0 |
| dry substance, g/100 g | 0.7 | 0.7 | 16.2 |
| conductivity mS/cm | 2.8 | 5.7 | 30.7 |
| L-fucose, % | 0.0 | 0.0 | 0.0 |
| galactose + glucose, % | 1.6 | 1.9 | 0.0 |
| lactose, % | 0.8 | 2.2 | 1.5 |
| 3-fucosyllactose, % | 0.3 | 1.6 | 56.4 |
| 2'fucosyllactose, % | 0.0 | 0.0 | 0.0 |
| difucosyllactose, % | 0.0 | 0.0 | 0.0 |

(continued)

| | Total permeate 1 | Total permeate 2 | Final concentrate |
|---|---|---|---|
| other, % | 97.4 | 94.3 | 42.1 |

The overall 3-FL yield calculated was 99.2% based on feed and permeate fractions, and salt removal calculated from conductivity was 69.2%. Sulfate removal was 31.7%, and phosphate removal was 64.1%. 617.0 kg of water was used, and, thus, the water-to-feed weight ratio normalized to a 4% (w/w) dry substance solution was 0.62 kg/kg (*i.e.,* the weight ratio of water to feed corresponds to 0.62 for a feed solution having a 4% (w/w) dry substance concentration). The conductivity retention was measured from 4 points, and the average value was 82.0%.

**Example 8**

**Membrane $MgSO_4$ and NaCl Retentions at Various Temperatures**

The process equipment included a plate and frame filtration unit (Alfa Laval Labstak M20), feed and diafiltration pump, heat exchanger, cooling unit, 100-liter feed tank as well as inlet and outlet pressure gauges and a pressure control valve. The total membrane area was 0.72 $m^2$. The tested membranes were NFG (Synder Filtration (Vacaville, CA)), TRISEP® UA60 (Microdyn-Nadir), NDX (Synder), TRISEP® XN45 (Microdyn-Nadir), 2-month used TRISEP® XN45 (also referred to as "XN45 (old)"), NF245 (DOW), NFW (Synder), Desal-5 DL (Suez), NFX (Synder) and Desal-5 DK (Suez). The term "XN45 (old)" refers to a TRISEP® XN45 membrane that has been used for 2 months in a nanofiltration unit at temperatures of from 5 to 10°C and pressures of from 10 to 30 bar, and has been exposed to several cleaning cycles.

Membranes DK, NFX, DL and NF245 are typically considered to be "tight" membranes because the $PO_4$ retention is greater than 65% at a pH greater than 5.0, and the $SO_4$ retention is greater than 97% at a pH greater than 5.0. The $MgSO_4$ retention, as specified by the manufacturer, is 99% (tight) for membrane DK, 99% (tight) for membrane NFX, 98-99% (tight) for membranes DL and NF245, 97% (open) for membrane NFW, 94-98% (open) for membrane XN45, 70-90% (very open) for membrane UA60, 90% (very open) for membrane NDX, and 50% (very open) for membrane NFG.

$MgSO_4$ retention test solution was made by adding 40 g $MgSO_4$ to 20 kg IEX water (2 g/l solution). pH was adjusted to 6.3 with a small amount of NaOH. The conductivity of feed was 2.25 mS/cm. NaCl retention test solution was made by adding 40 g NaCl to 20 kg IEX water (2 g/l solution). pH was adjusted to 6.2 with a small amount of NaOH. The conductivity of feed was 3.8 mS/cm. Retentions were calculated based on conductivity ($C_{MgSO4}$ = 0,0803*conductivity(mS/cm)$^{1.1618}$ and $C_{NaCl}$ = 0,0505*conductivity(mS/cm)$^{1.0352}$).

The feeds were heated in steps from 5-10°C to 60°C. The filtration trans membrane pressure was 7.5 Bar in temperatures 5-40 °C, 5.5 bar in 50°C and 4.5 bar in 60°C. The system was run in full recirculation and samples were taken in each temperature from all the membranes. The system was run in high crossflow so that liquid recovery was 5-20%.

$MgSO_4$ retention measured in temperature 25°C was 34.5%, 51.6%, 82.3%, 91.3%, 95.2%, 99.3%, 98.4%, 99.6%, 99.1% and 98.8% for the NFG, UA60, NDX, XN45 (old), XN45, NF245, NFW, DL, NFX and DK membranes, respectively. NaCl retention measured in temperature 25°C was 8.3%, 10.9%, 29.1%, 19.5%, 16.5%, 46.5%, 28.2%, 33.7%, 53.0% and 58.6% for the NFG, UA60, NDX, XN45 (old), XN45, NF245, NFW, DL, NFX and DK membranes, respectively.

$MgSO_4$ and NaCl retention change at different temperatures is shown in **Figures 1** and **2.**

**Example 9**

**HMO Model Solution NF Test in Cold Temperature**

The process equipment included a plate and frame filtration unit (Alfa Laval Labstak M20), feed and diafiltration pump, heat exchanger, cooling unit, 100-liter feed tank as well as inlet and outlet pressure gauges and a pressure control valve. The total membrane area was 0.72 $m^2$. The tested membranes were NFG (Synder), TRISEP® UA60 (Microdyn-Nadir), NDX (Synder), TRISEP® XN45 (Microdyn-Nadir), 2-month used TRISEP® XN45 (also referred to as "XN45 (old)"), NF245 (DOW), NFW (Synder), Desal-5 DL (Suez), NFX (Synder) and Desal-5 DK (Suez).

Model solution was made by dissolving 1.5 kg of 2'-FL powder, 0.2 kg of lactose, 0,2 kg of $MgSO_4$ and 0.2 kg of $KH_2PO_4$ to 7.9 kg of ion exchanged water.

10 kg of feed was fed into the 70-liter feed tank. The dry substance concentration in the feed was 20.7 g/100 g and the conductivity was 14.3 mS/cm. The pH was first adjusted to 6.3 with NaOH and then decreased using $H_2SO_4$

first to 5.2 and then to 3.4. The feed contained 13.55 g/100 g 2'-FL analyzed by HPLC and 5.8 g/kg $PO_4$ and 13.8 g/kg $SO_4$ analyzed by ion chromatography. Part of the added salt was lost due to precipitation, and, thus, not included in the analysis. Retention was calculated based on HPCL or ion chromatography analyses.

The feed was kept cool at 10.3-11.6°C. The filtration pressure was 40 Bar. The system was run in full recirculation, and samples were taken at each pH from all the membranes.

2'-FL retentions were increased significantly by decreasing pH from 6.3 to 5.2 and to 3.4. 2'-FL retention measured at a near-neutral pH of 6.3 was 88.6%, 96.0%, 96.7%, 98.6%, 99.1%, 98.3%, 98.9%, 99.0%, 98.7% and 97.8% for the NFG, UA60, NDX, XN45 (old), XN45, NF245, NFW, DL, NFX and DK membranes, respectively. 2'-FL retention measured in low pH of 3.4 was 90.8%, 99.3%, 98.6%, 99.1%, 99.6%, 99.2%, 99.2%, 99.5%, 99.3% and 98.9% for the NFG, UA60, NDX, XN45 (old), XN45, NF245, NFW, DL, NFX and DK membranes, respectively.

Conductivity retentions were defined at pH's of 6.3, 5.2 and 3.4. The conductivity retention measured at a near-neutral pH of 6.3 was 19.2%, 35.2%, 81.3%, 75.1%, 79.4%, 89.5%, 89.9%, 93.9%, 94.5% and 92.4% for the NFG, UA60, NDX, XN45 (old), XN45, NF245, NFW, DL, NFX and DK membranes, respectively. The conductivity retention measured at a low pH of 3.4 was -27.9%, -4.1%, 70.5%, 15.8%, 20.8%, 63.2%, 40.1%, 67.1%, 71.5 and 77.0% for the NFG, UA60, NDX, XN45 (old), XN45, NF245, NFW, DL, NFX and DK membranes, respectively.

Phosphate retentions were defined at pH's of 6.3, 5.2 and 3.4. Phosphate retention measured at a near-neutral pH of 6.3 was -6.5%, -5.8%, 76.3%, 52.5%, 54.7%, 77.8%, 82.4%, 91.2%, 95.1% and 97.6% for the NFG, UA60, NDX, XN45 (old), XN45, NF245, NFW, DL, NFX and DK membranes, respectively. Phosphate retention measured at a low pH of 3.4 was 32.12%, 13.2%, 49.7%, 23.4%, 32.0%, 59.7%, 80.1%, 47.6%, 61.9% and 65.0% for the NFG, UA60, NDX, XN45 (old), XN45, NF245, NFW, DL, NFX and DK membranes, respectively.

Sulfate retentions were defined at pH's of 6.3, 5.2 and 3.4. Sulfate retention measured at a near-neutral pH of 6.3 was 59.5%, 73.4%, 94.7%, 94.1%, 95.9%, 97.8%, 98.0%, 98.8%, 98.3% and 97.2% for the NFG, UA60, NDX, XN45 (old), XN45, NF245, NFW, DL, NFX and DK membranes, respectively. Sulfate retention measured at a low pH of 3.4 was 31.5%, 54.8%, 93.6%, 66.6%, 70.8%, 89.7%, 80.3%, 92.6%, 92.9% and 95.5% for the NFG, UA60, NDX, XN45 (old), XN45, NF245, NFW, DL, NFX and DK membranes, respectively.

## Example 10

### Cold Temperature Concentration and Salt Removal of 2'-FL Liquid with Very Open NF Membranes Operated in Low pH

The process equipment included a plate and frame filtration unit (Alfa Laval Labstak M20), feed and diafiltration pump, heat exchanger, cooling unit, 100-liter feed tank as well as inlet and outlet pressure gauges and a pressure control valve. The total membrane area was 0.72 $m^2$. The tested membranes were TRISEP® UA60 (Microdyn-Nadir).

Ultrafiltration permeate from an *E. Coli* based fermentation of 2'-FL was used as a feed. The aim was to concentrate 2'-FL contained in the feed while passing impurities (salts, monomeric sugars, dimeric sugars) to the permeate.

72 kg of feed was fed into a 100-liter feed tank. The dry substance concentration in the feed was 3.1 g/100 g, the conductivity was 6.0 mS/cm and the pH was 7.2. pH was decreased to 3.9 using 1.0 kg of 80% acetic acid. Permeate was collected from all the membranes. The feed composition is shown in **Table 10-1,** whereby the HPLC analyses are given on a percent dry substance basis.

**TABLE 10-1**

| Feed Composition (%) | |
|---|---|
| L-fucose | 0.0 |
| galactose + glucose | 0.9 |
| lactose | 10.9 |
| 3-fucosyllactose | 0 |
| 2' fucosyllactose | 49.9 |
| difucosyllactose | 4.6 |
| other | 33.7 |

The feed was kept cool at 5-11°C, and water was used for diafiltration. The filtration pressure was 10-40 bar, and the concentrate DS concentration was controlled to keep flux above 6 kg/$m^2$/hr (the minimum flux point observed).

After batch filtration, two permeate fractions and a final concentrate fraction were collected. The results, including HPLC analyses on a percent dry substance basis for the permeate two fractions and final concentrate, are shown in **Table 10-2.**

**TABLE 10-2**

| | Total permeate 1 | Total permeate 1 | Final concentrate |
|---|---|---|---|
| mass, kg | 48.5 | 34.5 | 10.5 |
| dry substance, g/100 g | 1.2 | 1.1 | 14.1 |
| conductivity mS/cm | 5.5 | 3.8 | 1.8 |
| L-fucose, % | 0.0 | 0.0 | 0.0 |
| galactose + glucose, % | 1.1 | 2.0 | 0.4 |
| lactose, % | 0.5 | 4.0 | 13.7 |
| 3-fucosyllactose, % | 0.0 | 0.0 | 0.0 |
| 2'fucosyllactose, % | 0.0 | 10.2 | 65.7 |
| difucosyllactose, % | 0.0 | 0.0 | 6.3 |
| other, % | 98.4 | 83.8 | 13,9 |

The overall 2'-FL yield calculated was 96.5%, and salt removal calculated from conductivity was 92.6 - 95.6%. 21.5 kg of water was used, and, thus, the water-to-feed weight ratio normalized to a 4% (w/w) dry substance solution was 0.39 kg/kg (*i.e.,* the weight ratio of water to feed corresponds to 0.39 for a feed solution having a 4% (w/w) dry substance concentration). The conductivity retention average measured from 5 sample points was 21.7%. 2'-FL retention was measured from 4 points, and the average value was 99.2%

The words "comprise", "comprises" and "comprising" are to be interpreted inclusively rather than exclusively. This interpretation is intended to be the same as the interpretation that these words are given under United States patent law at the time of this filing.

The singular forms "a" and "an" are intended to include plural referents unless the context dictates otherwise. Thus, for example, a reference to the presence of "an excipient" does not exclude the presence of multiple excipients unless the context dictates otherwise.

All references cited in this specification are incorporated by reference into this specification.

## Claims

**1.** A process for preparing a purified human milk oligosaccharide (HMO), wherein:

the process comprises:

- feeding an HMO solution into a nanofiltration unit,
- filtrating the HMO solution using the nanofiltration unit to produce a concentrate and a permeate, and
- collecting the concentrate;

the HMO solution comprises an aqueous medium comprising an HMO to be purified and a second carbohydrate; the nanofiltration unit comprises a membrane having:

- an $MgSO_4$ retention of at least about 50% (at 25°C, 2 g/l concentration, 8 bar and pH 6-7), and
- an NaCl retention of up to about 60% (at 25°C, 2 g/l concentration, 8 bar and pH 6-7); and

the filtrating is carried out at a temperature of less than 10°C.

**2.** A process for preparing a human milk oligosaccharide (HMO), wherein:

the process comprises:

- feeding an HMO solution into a nanofiltration unit,
- filtrating the HMO solution using the nanofiltration unit to produce a concentrate and a permeate, and
- collecting the concentrate;

the HMO solution comprises an aqueous medium comprising an HMO to be purified and a second carbohydrate; the nanofiltration unit comprises a membrane having:

- an $MgSO_4$ retention of greater than 90% (at 25°C, 2 g/l concentration, 8 bar and pH 6-7), and
- an NaCl retention of up to about 60% (at 25°C, 2 g/l concentration, 8 bar and pH 6-7); and

the filtrating is carried out at a temperature of no greater than about 18°C.

**3.** A process for preparing a purified human milk oligosaccharide (HMO), wherein:

the process comprises:

- feeding an HMO solution into a nanofiltration unit,
- filtrating the HMO solution using the nano filtration unit to produce a concentrate and a permeate, and
- collecting the concentrate;

the HMO solution comprises an aqueous medium comprising an HMO to be purified and a second carbohydrate; the nanofiltration unit comprises a membrane having:

- an $MgSO_4$ retention of at least about 50% (at 25°C, 2 g/l concentration, 8 bar and pH 6-7), and
- an NaCl retention of up to about 60% (at 25°C, 2 g/l concentration, 8 bar and pH 6-7);

the nanofiltration unit comprises a membrane having a molecular weight cut off of less than 600 Dalton; and the filtrating is carried out at a temperature of no greater than about 18°C.

**4.** The process according to any one of the preceding claims, wherein:

the filtrating is carried out at a temperature of less than 10°C; and the nanofiltration unit comprises a membrane having:

- an $MgSO_4$ retention of greater than 90% (at 25°C, 2 g/l concentration, 8 bar and pH 6-7), and
- a molecular weight cut off of less than 600 Dalton.

**5.** The process according to any one of the preceding claims, wherein the membrane is characterized as exhibiting a retention of the HMO to be purified of at least 96.0% when operating under the following conditions:

- a flux of from 5 to 20 $kg/m^2/hr$, and
- a concentration of the HMO to be purified in the HMO solution of from 50 to 300 g/L.

**6.** The process according to any one of the preceding claims, wherein:

the process comprises feeding into the nanofiltration unit water for diafiltration in addition to the HMO solution, and the weight ratio of the diafiltration water to the HMO solution is less than 1.6 when normalized to an HMO solution having a dry substance composition of 4% (w/w).

**7.** The process according to claim 6, wherein:

the weight ratio of the diafiltration water to the HMO solution is from about 0.09 to 0.3 when normalized to an HMO solution having a dry substance composition of 4% (w/w), the nanofiltration provides a salt removal of at least about 40%, and the nanofiltration provides a yield of the HMO to be purified of at least about 85%.

**8.** The process according to claim 6, wherein:

the weight ratio of the diafiltration water to the HMO solution is from 0.3 to 0.6 when normalized to an HMO solution having a dry substance composition of 4% (w/w),
the nanofiltration provides a salt removal of at least about 90%, and
the nanofiltration provides a yield of the HMO to be purified of at least about 90%.

**9.** The process according to claim 6, wherein:

the weight ratio of the diafiltration water to the HMO solution is from 0.6 to 0.9 when normalized to an HMO solution having a dry substance composition of 4% (w/w),
the nanofiltration provides a salt removal of at least 68%, and
the nanofiltration provides a yield of the HMO to be purified of at least about 85%.

**10.** The process according to any one of the preceding claims, wherein the membrane has a lactose retention of at least about 80% (at 25°C, 20g/L concentration, flux 5-20 kg/$m^2$/h, and pH 8).

**11.** The process according to any one of the preceding claims, wherein the HMO solution comprises or is derived from a product of a fermentation process.

**12.** The process according to any one of the preceding claims, wherein the HMO to be purified is 2'-fucosyllactose.

**13.** The process according to any one of claims 1-11, wherein HMO to be purified is 3-fucosyllactose.

**14.** The process according to any one of the preceding claims, wherein the filtrating is carried out at a pH of from about 3.0 to about 5.5.

FIG. 1

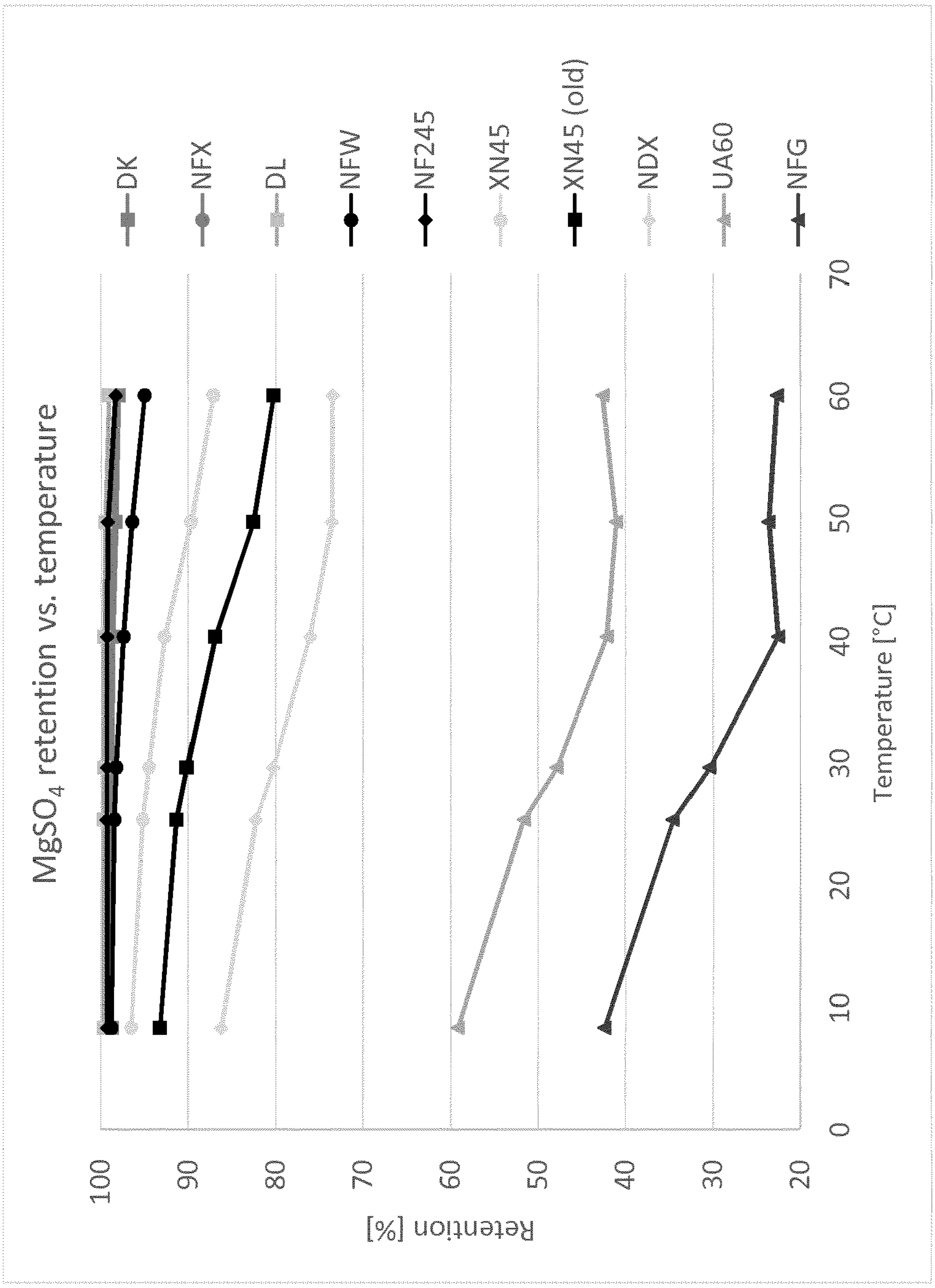
MgSO4 retention vs. temperature
Retention [%]
100
90
80
70
60
50
40
30
20
Temperature [°C]
0
10
20
30
40
50
60
70
DK
NFX
DL
NFW
NF245
XN45
XN45 (old)
NDX
UA60
NFG

FIG. 2

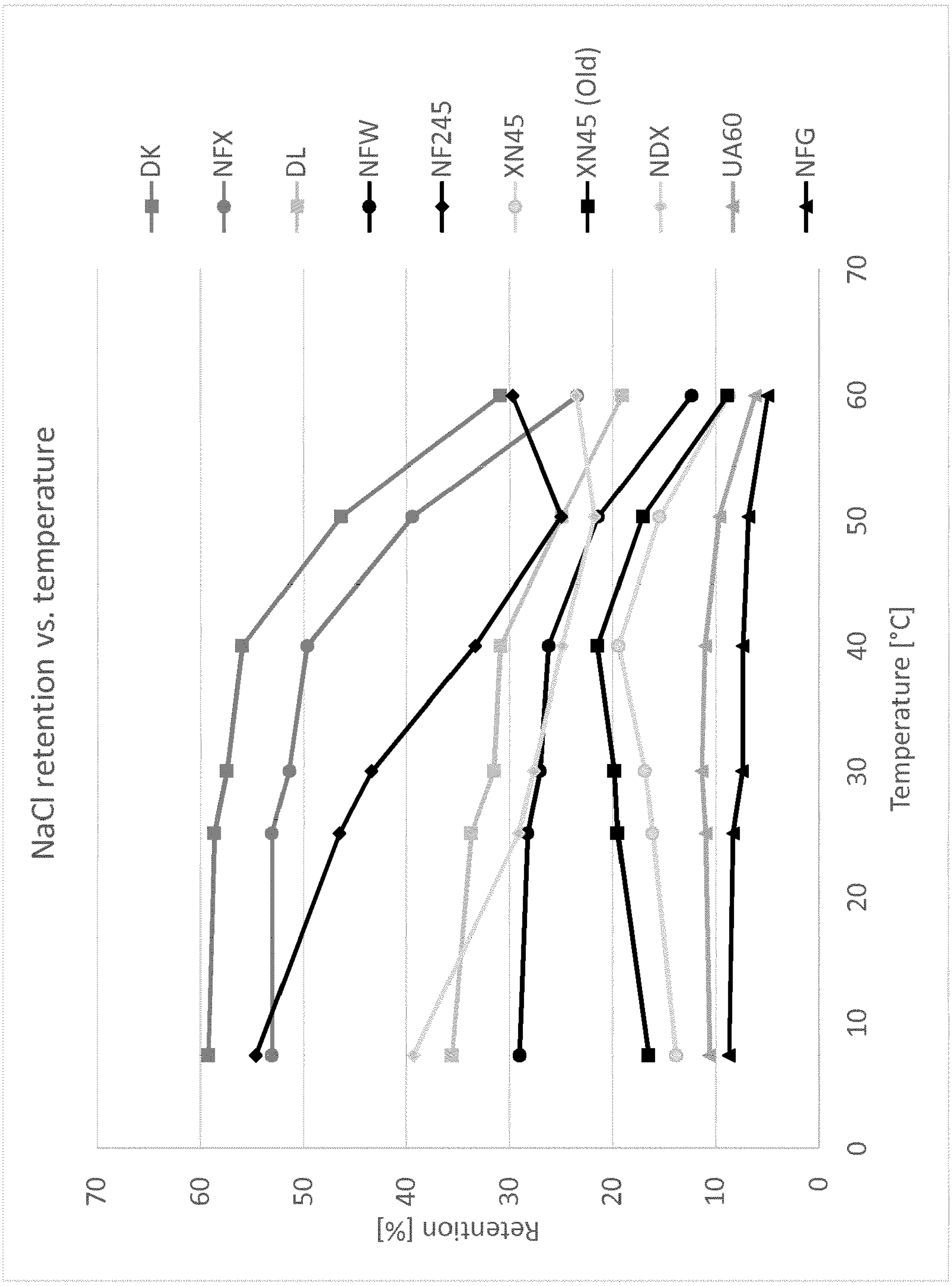
NaCl retention vs. temperature
Retention [%]
Temperature [°C]
0
10
20
30
40
50
60
70
DK
NFX
DL
NFW
NF245
XN45
XN45 (Old)
NDX
UA60
NFG

Europäisches Patentamt
European Patent Office
Office européen des brevets

# EUROPEAN SEARCH REPORT

Application Number
EP 19 15 6345

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/003133 A1 (GLYCOM AS [DK]) 3 January 2019 (2019-01-03) * the whole document * * claims * * In particular page 8, line 17 to page 9, line 9 * ----- | 1-14 | INV. C07H1/06 C07H1/08 C07H3/06 A23L5/00 A23L29/30 B01D61/02 |
| Y | WO 2017/220697 A1 (ARLA FOODS AMBA [DK]) 28 December 2017 (2017-12-28) * page 18, line 25 - page 21, line 5 * ----- | 1-14 | |
| Y | WO 98/15581 A1 (CYTEL CORP [US]; DEFREES SHAWN [US]) 16 April 1998 (1998-04-16) * abstract * * page 7, line 9 - page 10, line 3 * * examples 5-8 * ----- | 1-14 | |
| Y | GOULAS A K ET AL: "Purification of oligosaccharides by nanofiltration", JOURNAL OF MEMBRANE SCIENCE, ELSEVIER BV, NL, vol. 209, no. 1, 1 November 2002 (2002-11-01), pages 321-335, XP004382364, ISSN: 0376-7388, DOI: 10.1016/S0376-7388(02)00362-9 * abstract * * Part 3.2. Membranes; pages 323-324; table 1 * ----- | 1-14 | TECHNICAL FIELDS SEARCHED (IPC) C07H B01D A23L |
| | -/-- | | |

The present search report has been drawn up for all claims

| Place of search | Date of completion of the search | Examiner |
|---|---|---|
| Munich | 5 September 2019 | Gohlke, Pascale |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 15 6345

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NORDVANG RUNE T ET AL: "Separation of 3'-sialyllactose and lactose by nanofiltration: A trade-off between charge repulsion and pore swelling induced by hig", SEPARATION AND PURIFICATION TECHNOLOGY, ELSEVIER SCIENCE, AMSTERDAM, NL, vol. 138, 22 October 2014 (2014-10-22), pages 77-83, XP029101940, ISSN: 1383-5866, DOI: 10.1016/J.SEPPUR.2014.10.012 * the whole document * ----- | 1-14 | |

TECHNICAL FIELDS SEARCHED (IPC)

The present search report has been drawn up for all claims

| Place of search | Date of completion of the search | Examiner |
|---|---|---|
| Munich | 5 September 2019 | Gohlke, Pascale |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

## ANNEX TO THE EUROPEAN SEARCH REPORT ON EUROPEAN PATENT APPLICATION NO. EP 19 15 6345

This annex lists the patent family members relating to the patent documents cited in the above-mentioned European search report. The members are as contained in the European Patent Office EDP file on
The European Patent Office is in no way liable for these particulars which are merely given for the purpose of information.

05-09-2019

| Patent document cited in search report | Publication date | Patent family member(s) | Publication date |
|---|---|---|---|
| WO 2019003133 A1 | 03-01-2019 | WO 2019003133 A1 | 03-01-2019 |
| | | WO 2019003135 A1 | 03-01-2019 |
| | | WO 2019003136 A1 | 03-01-2019 |
| WO 2017220697 A1 | 28-12-2017 | AR 108854 A1 | 03-10-2018 |
| | | AU 2017281328 A1 | 17-01-2019 |
| | | BR 112018076740 A2 | 26-03-2019 |
| | | CA 3028529 A1 | 28-12-2017 |
| | | CL 2018003785 A1 | 14-06-2019 |
| | | CN 109561724 A | 02-04-2019 |
| | | EA 201892770 A1 | 31-07-2019 |
| | | EP 3471561 A1 | 24-04-2019 |
| | | JP 2019518462 A | 04-07-2019 |
| | | KR 20190031482 A | 26-03-2019 |
| | | PH 12018502695 A1 | 08-04-2019 |
| | | WO 2017220697 A1 | 28-12-2017 |
| WO 9815581 A1 | 16-04-1998 | AT 304546 T | 15-09-2005 |
| | | AU 735695 B2 | 12-07-2001 |
| | | CA 2268168 A1 | 16-04-1998 |
| | | DE 69734205 D1 | 20-10-2005 |
| | | DE 69734205 T2 | 06-07-2006 |
| | | DK 0931097 T3 | 16-01-2006 |
| | | EP 0931097 A1 | 28-07-1999 |
| | | HU 0001634 A2 | 28-09-2000 |
| | | IL 129363 A | 12-02-2003 |
| | | IL 149275 A | 04-07-2007 |
| | | JP 2001502005 A | 13-02-2001 |
| | | KR 20000049057 A | 25-07-2000 |
| | | NZ 335203 A | 27-10-2000 |
| | | US 6454946 B1 | 24-09-2002 |
| | | US 2002148791 A1 | 17-10-2002 |
| | | US 2003029799 A1 | 13-02-2003 |
| | | US 2005269265 A1 | 08-12-2005 |
| | | WO 9815581 A1 | 16-04-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018164937 A